# EUROPEAN PATENT SPECIFICATION

(11) **EP 2 430 972 B1**
(45) Date of publication and mention of the grant of the patent: **29.11.2017**
(21) Application number: 10834453.2
(22) Date of filing: 27.10.2010
(51) Int. Cl.: A61B 1/04, A61B 1/00, G02B 23/24, A61B 1/227, A61B 1/233

(54) **HAND-HELD WIRELESS ENDOSCOPE**
TRAGBARES DRAHTLOSES ENDOSKOP
ENDOSCOPE SANS FIL PORTATIF

(30) Priority: 04.12.2009 JP 2009276593
(43) Date of publication of application: 21.03.2012
(73) Proprietor: Olympus Corporation, Tokyo 192-8507 (JP)
(72) Inventor: OGAWA, Tomoaki, Tokyo 192-8512 (JP); SUZUKI, Takeo, Tokyo 192-8512 (JP)
(74) Representative: Gunzelmann, Rainer
(86) International application number: PCT/JP2010/069032
(87) International publication number: WO 2011/068000

(56) References cited:
- EP-A1- 0 868 878
- WO-A1-2009/020724
- JP-A- 2002 017 668
- JP-A- 2007 029 718
- US-A1- 2007 185 379
- US-A1- 2009 066 585
- US-A1- 2009 295 648

## Description

### Technical Field

The present invention relates to a handheld endoscope capable of performing wireless communication, which is operated when an operator (practitioner) holds a gripping section of an endoscope main body.

### Background Art

When using an endoscope, especially a flexible scope, an operator often holds a gripping section (grip) of an endoscope main body (operation section) and uses the endoscope with the endoscope main body being erected to provide the longitudinal direction in the substantially vertical direction. However, in an otologic (otorhinolaryngologic) case, it is general to conduct a procedure with a patient being seated. Therefore, when the endoscope main body is used in the upright posture to provide the longitudinal direction in the substantially vertical direction, an insertion section extending from a lower portion of the endoscope main body in the substantially lower direction is greatly bent so that a distal end portion of the insertion section faces a front side of the patient's face. Accordingly, in the department of otology, the endoscope may be used in a state in which the endoscope main body is laid down horizontally, the insertion section is extended in the substantially horizontal direction without greatly bending, and the insertion section is inserted into a nasal cavity with the distal end portion of the insertion section facing the front side of the patient's face.

When using the endoscope for otologic cases as described above, to further improve the operability, it can be considered that such an endoscope having an operation section itself formed into a gun type (pistol) shape such as disclosed in a patent literature 1 or a patent literature 2 is used for otologic cases and an insertion section is extended in the substantially horizontal direction with respect to the front side of a patient's face and then inserted, thereby comfortably operating the endoscope.

Furthermore, as an endoscope, there is a so-called "wireless endoscope" like that disclosed in Patent Jpn. Pat. Appln. KOKAI Publication No. 2005-323889 or Jpn. Pat. Appln. KOKAI Publication No. H11-155815, the endoscope having the operability in treatments being improved by wirelessly transmitting an endoscopic image acquired by an imaging element to a processing device without a cable. Since an otologic endoscope no longer requires the cable either, the wireless endoscope is very effective since it does not obstruct an operation in a small examination room.

Moreover, like the patent literature 2 or a patent literature 3, if a wireless circuit is mounted in an endoscope whose endoscope main body (operation section) is of a gun type, the operability of the endoscope for otology and others can be greatly improved.

### Citation List

### Patent Literature

Patent Literature 1: Jpn. Pat. Appln. KOKAI Publication No. H06-235867
Patent Literature 2: Jpn. Pat. Appln. KOKAI Publication No. 2005-323889
Patent Literature 3: Jpn. Pat. Appln. KOKAI Publication No. H11-155815
Document US2007/185379 discloses a remote inspection device comprising a digital imager for use during endoscopy or boroscopy procedures

### Summary of Invention

It is desirable that an antenna (a communication antenna) which wirelessly communicates an endoscopic image converted to a wireless signal to a processing device is kept away from electric wave inhibitors such as a human body, a metallic component, or an electronic component as much as possible.

In case of an endoscope having the above-described gun type shape, at least a part of a main body of the endoscope is constituted of a metallic component. When arranging the communication antenna in the main body of the endoscope, arranging the communication antenna in a portion having an electronic component or a metallic frame or a portion at which an operator holds the endoscope (a gripping portion) affects the directionality of the antenna to deteriorate wireless communication performance, and the directionality that is uniform in the circumference may not be possibly obtained. Therefore, ingenuity must be exercised in the arrangement of the communication antenna to prevent the electric wave from being inhibited.

However, the patent literature 1 does not have an idea of applying the endoscope having the gun type shape to the wireless endoscope. In the patent literature 2, although there is a description concerning the arrangement of the gripping section and the communication antenna, the arrangement of the communication antenna, electronic components, and others assuming an improvement in the wireless communication performance with respect to the outside is not considered. Further, in the patent literature 3, although an example that a communication radiofrequency antenna is mounted in an endoscope having the gun type shape is described, it cannot be said that the arrangement of the communication antenna takes an improvement in the wireless communication performance with respect to the outside into consideration.

Thus, it is an object of the present invention to provide an endoscope which has not only improved gripping properties/operability but also reduced influence of a human body of an operator who holds an endoscope main body or a metallic body in the endoscope main body including an electric circuit on directionality of an antenna in wireless communication and further has improved wireless communication performance.

An endoscope of the invention includes: an insertion section which is configured to be extended in a front-and-back direction, which is configured to be inserted into a body cavity, and which includes an observation optical system configured to image an inside of the body cavity; an endoscope main body which is provided on a rear end side of the insertion section and which includes a gripping section gripped by an operator; a first antenna which is arranged at a position closer to the insertion section than the gripping section; and a second antenna which is arranged at a position farther from the insertion section than the gripping section, wherein an image obtained by imaging the inside of the body cavity by the observation optical system is converted into a wireless signal, and transmission/reception of the wireless signal to/from the outside is enabled through the first and/or second antenna.

### Brief Description of Drawings

The accompanying drawings, which are incorporated in and constitute a part of the specification, illustrate embodiments of the invention, and together with the general description given above and the detailed description of the embodiments given below, serve to explain the principles of the invention.
FIG. 1 is a schematic view showing an entire configuration of an endoscopic system according to one embodiment of the present invention;
FIG. 2 is a schematic left side view showing an appearance of an endoscope in the endoscopic system according to one embodiment;
FIG. 3 is a schematic view showing an internal configuration of the endoscope in the endoscopic system according to one embodiment;
FIG. 4A is schematic view showing an appearance of the endoscope in the endoscopic system according to one embodiment when an effective length of an insertion section differs;
FIG. 4B is schematic view showing an appearance of the endoscope in the endoscopic system according to one embodiment when an effective length of an insertion section differs;
FIG. 5 is a schematic view showing an appearance of the endoscope having an attachment disposed to an insertion section in the endoscopic system according to one embodiment;
FIG. 6 is a schematic view showing an appearance of the endoscope in the endoscopic system according to one embodiment;
FIG. 7 is a schematic showing a state in which an operator holds the endoscope in the endoscopic system according to one embodiment;
FIG. 8A is a schematic view showing a state in which a distal end portion of the insertion section of the endoscope is inserted into a nasal cavity in the endoscopic system according to one embodiment;
FIG. 8B is a schematic view showing a state in which the distal end portion of the insertion section of the endoscope is inserted into an aural cavity in the endoscopic system according to one embodiment;
FIG. 9A is a schematic view showing a state in which an outer sheath cover is disposed to a bending operation section of a curved section of the endoscope in the endoscopic system according to one embodiment;
FIG. 9B is a schematic view showing the bending operation section in a state in which the outer sheath cover is removed in the endoscopic system according to one embodiment;
FIG. 9C is a schematic perspective view showing a state in which a frame in FIG. 9B is seen from a back surface side in the endoscopic system according to one embodiment;
FIG. 10 is a schematic view of a conventional endoscopic system showing a state in which an outer sheath cover of an endoscope is fixed;
FIG. 11A shows a schematic view of a cylindrical body of a base section of an endoscope main body of an endoscope, and a second operation switches (function changeover switches) arranged on a front side in an endoscopic system according to one embodiment;
FIG. 11B shows a schematic view of a cylindrical body of a base section of an endoscope main body of an endoscope, and a third operation switch (power supply switch) arranged on a rear side in an endoscopic system according to one embodiment;
FIG. 12 is a schematic perspective view showing first operation switches provided at a boundary part of the base section, a curved section, and a head section of the endoscope main body of the endoscope in the endoscopic system according to one embodiment;
FIG. 13A shows a state in which the first operation switch depicted in FIG. 12 is disposed, and is a schematic view showing a state in which an outer sheath is removed;
FIG. 13B shows a state in which the first operation switch depicted in FIG. 12 is disposed, and is an enlarged schematic view showing the first operation switch surrounded by a broken line in FIG. 13A;
FIG. 14A is a schematic view showing a substrate unit arranged in the base section of the endoscope main body of the endoscope in the endoscopic system according to one embodiment;
FIG. 14B is a schematic view showing the substrate unit removed from the base section in the endoscopic system according to one embodiment;
FIG. 14C is a schematic view showing a state in which an outer sheath at the boundary part of the base section, the curved section, an the front side of the head section of the endoscope main body is removed in the endoscopic system according to one embodiment;
FIG. 15 is a schematic view showing a peripheral region of an inner frame arranged in the base section of the endoscope main body of the endoscope in the endoscopic system according to one embodiment;
FIG. 16 is a schematic view showing a state in which a light source unit is arranged in the bending operation section of the endoscope main body of the endoscope in the endoscopic system according to one embodiment;
FIG. 17A is a schematic view showing the arrangement of an electric system such as a substrate unit, a battery, antennas, a light source unit, and others with respect to the endoscope in the endoscopic system according to one embodiment;
FIG. 17B is a schematic view showing a direction along which a first antenna is arranged at a part denoted by reference number 40a in FIG. 17A and an orientation direction of the antenna in the endoscopic system according to one embodiment;
FIG. 17C is a schematic view showing a direction along which a second antenna is arranged at a part denoted by reference number 40b in FIG. 17A and an orientation direction of the antenna in the endoscopic system according to one embodiment;
FIG. 18 is a schematic view showing a bracket arranged in the substrate unit arranged in the endoscope main body of the endoscope in the endoscopic system according to one embodiment;
FIG. 19A is a schematic perspective view showing a state in which the cylindrical body is disposed with the substrate unit being arranged in the endoscope main body of the endoscope in the endoscopic system according to one embodiment;
FIG. 19B is a schematic view of FIG. 19A in the endoscopic system according to one embodiment;
FIG. 19C is a schematic view showing a state in which a rigid substrate depicted in FIG. 19B can be inclined to attach the cylindrical body to the substrate unit in the endoscopic system according to one embodiment;
FIG. 20 is a front view showing a display device that displays an endoscopic image of the endoscopic system according to one embodiment;
FIG. 21 is a schematic perspective view showing a state in which the battery can be attached to/detached from a battery accommodation section formed in the base section of the endoscope main body of the endoscope in the endoscopic system according to one embodiment;
FIG. 22A is a schematic view showing the battery arranged in the battery accommodation section of the endoscope in the endoscopic system according to one embodiment;
FIG. 22B is a schematic view showing a state in which the battery depicted in FIG. 22A is observed from the opposite side and a state in which the battery expands in the endoscopic system according to one embodiment;
FIG. 23 is a schematic view showing a state in which a setting signal write instrument arranged in the battery accommodation section of the endoscope in the endoscopic system according to one embodiment is connected to a write device;
FIG. 24A is a schematic view showing a state in which the setting signal write instrument is accommodated in the battery setting portion of the endoscope in the endoscopic system according to one embodiment;
FIG. 24B is a schematic cross-sectional view showing a state in which the setting signal write instrument is disposed in the endoscopic system according to one embodiment;
FIG. 25A is a schematic perspective view showing the setting signal write instrument arranged in the battery accommodation section of the endoscope in the endoscopic system according to one embodiment;
FIG. 25B is a schematic perspective view showing the battery arranged in the battery accommodation section;
FIG. 26 is a schematic view showing a processing device of the endoscopic system according to one embodiment;
FIG. 27 is a schematic view sowing a state in which the antennas are disposed to the processing device of the endoscopic system according to one embodiment; and
FIG. 28 is a schematic perspective view showing a back surface of a housing of the processing device of the endoscopic system according to one embodiment.

### Brief Description of Embodiments

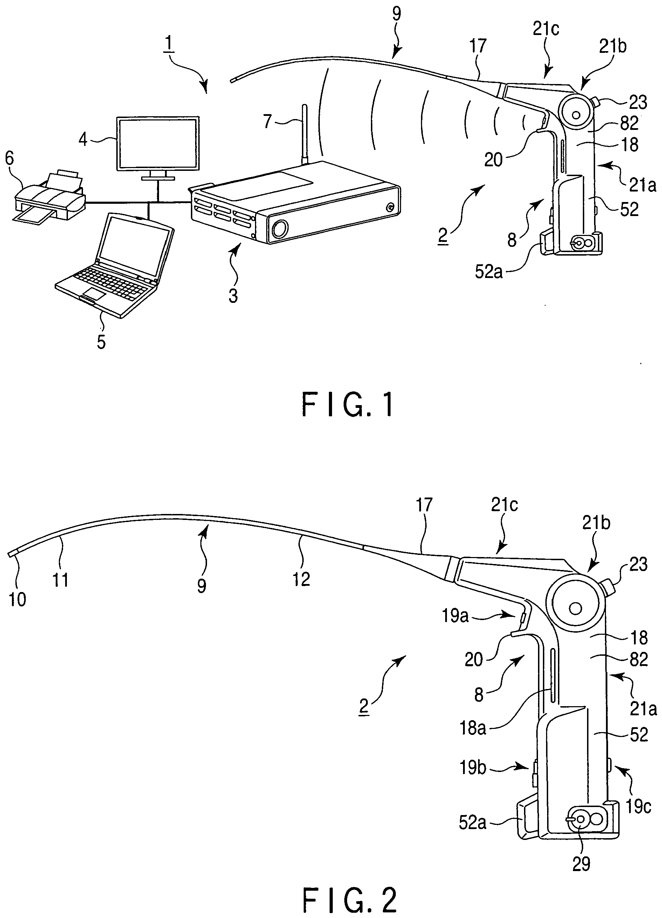

A mode for carrying out the present invention will now be described hereinafter with reference to FIG. 1 to FIG. 28.

Here, a description will be given as to an example of an endoscopic system (a wireless endoscopic system) 1 that includes a vertically long substrate unit 30 including an electric substrate having electronic components such as a wireless communication circuit 39, an image processing circuit 38, wireless communication antennas 40a and 40b which will be described later, converts an endoscopic image into a wireless signal by using the wireless communication circuit 39 to be wirelessly transmitted to a processing device 3 and the like, and displays the endoscopic image in, e.g., an external graphic display device 4.

As shown in FIG. 1, the endoscopic system 1 according to the embodiment includes an endoscope 2, a processing device 3 which receives a wireless signal transmitted from the endoscope 2 and converts the wireless signal into a picture signal, and a display device 4 which displays the picture signal generated by the processing device 3 as a picture. It is to be noted that the processing device 3 may be connected to the display device 4 through, e.g., a cord, or wireless communication may be enabled between the processing device 3 and the display device 4.

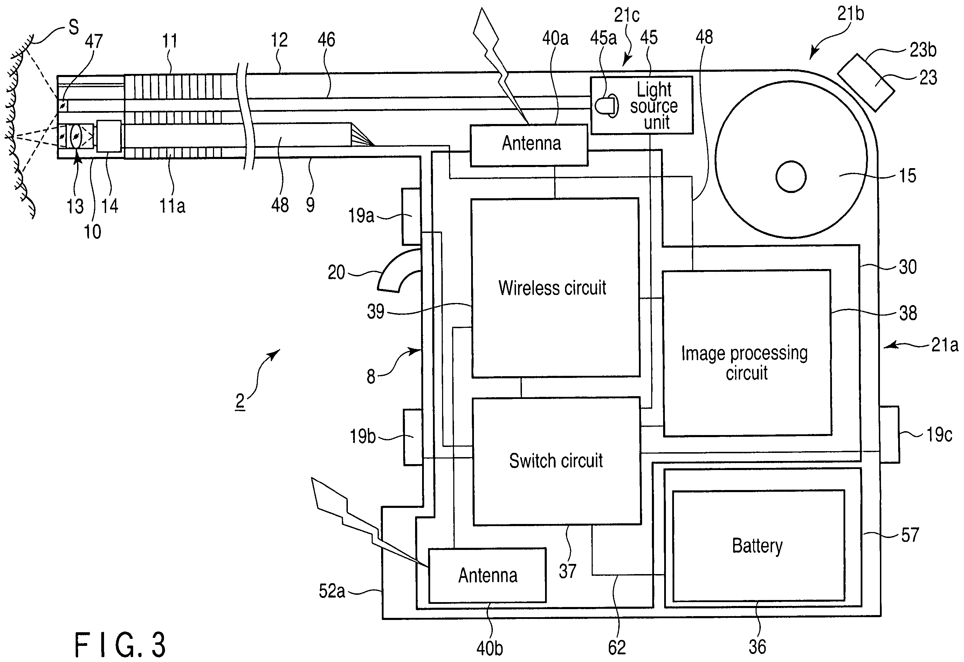

As shown in FIG. 3, the endoscope 2 includes a later-described image processing circuit 38 which converts an acquired picture (endoscopic image) into a wireless signal. When the wireless signal is transmitted from transmission antennas 40a and 40b through a later-described wireless circuit 39 which is built in the endoscope 2 and connected to the image processing circuit 38, the wireless signal is received by reception antennas 7 connected to the processing device 3. The processing device 3 converts the wireless signal into a picture signal and performs image processing to the picture signal. The picture signal output from the processing device 3 is displayed as a picture in a screen of the display device 4.

The endoscopic system 1 may include a computer 5 or a printing device 6. In this case, the computer 5 or the printing device 6 is connected to the processing device 3. For example, the computer 5 has a function of appropriately setting circuits (electronic substrates) 37, 38, and 39 on a substrate unit 30 of the endoscope 2 or first to third operation switches 19a, 19b, and 19c or storing or analyzing the picture signal generated by the processing device 3. The printing device 6 has a function of printing a still image fetched from the picture signal generated by the processing device 3 or a document created by the computer 5.

The endoscope 2 according to the embodiment will now be described with reference to FIG. 2 to FIG. 21.

As shown in FIG. 2 and FIG. 3, the endoscope 2 includes an endoscope operation section (endoscope main body) 8 which is gripped by a user and configured to perform operations and an insertion section 9 which is extended from the endoscope operation section 8 in a front-and-back direction (long axis direction) from a front side (distal end side) toward a back side (proximal end side) and inserted into a body cavity. In other words, in the endoscope 2, an upper end portion of the endoscope operation section 8 that is long in the vertical direction is connected to a rear end portion (proximal end portion) of the insertion section 9 extended in the front-and-back direction.

As shown in FIG. 2, the insertion section 9 includes a distal end hard section 10, a bending section 11 that is operated to bend, and a long flexible tube section 12 having flexibility in an order from the front side toward the back side. That is, the bending section 11 is continuously provided to a rear end portion of the distal end hard section 10 and the flexible tube section 12 is continuously provided to a rear end portion of the bending section 11. It is to be noted that a rear end portion of the flexible tube section 12 (rear end portion of insertion section 9) is continuously connected to a distal end portion of a head section 21c of the endoscope operation section 8 through a protection hood 17.

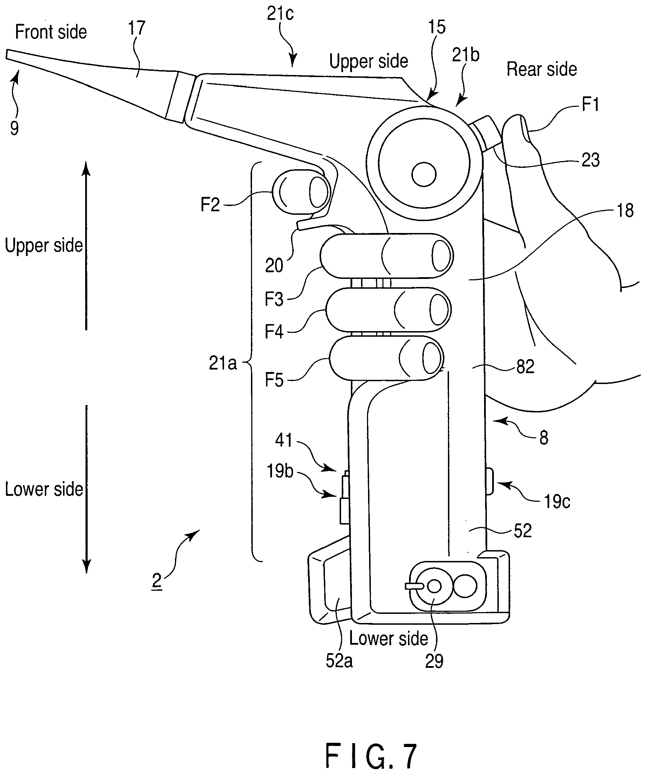

A length of the insertion section 9 of the endoscope 2 according to the embodiment can be appropriately set. In case of using the endoscope 2 for the otorhinolaryngology in particular, the insertion section 9 excellently follows precipitous movements of a patient when the insertion section 9 of the endoscope 2 is inserted into an affected area. However, in case of using the endoscope 2 for a medical examination of an anterior portion of the nasal cavity, an ear, and the like, handing or manipulation of the insertion section 9 is complicated if the insertion section 9 is very flexible and has a long effective length. Therefore, it is preferable for the insertion section 9 of the endoscope 2 according to the embodiment to have not only pliableness by adjusting flexibility of the flexible tube section 12 but also flexibility that enables the distal end hard section 10 of the insertion section 9 to be arranged at a position apart from the endoscope operation section 8 as shown in FIG. 2. Further, when the effective length of the insertion section 9 is reduced, hand movement of an operator can be suppressed. Therefore, according to the endoscope 2 of the embodiment, when the endoscope 2 is gripped as shown in FIG. 7, the medical examination in the otorhinolaryngology and others can be carried out with the endoscope 2 being held while maintaining a natural posture. As described above, since the insertion section 9 is extended in the front-and-back direction and the endoscope operation section (endoscope main body) 8 is provided at the rear end of the insertion section 9, the gripping properties/operability of the endoscope 2 in use for a treatment in the otorhinolaryngology and others can be improved.

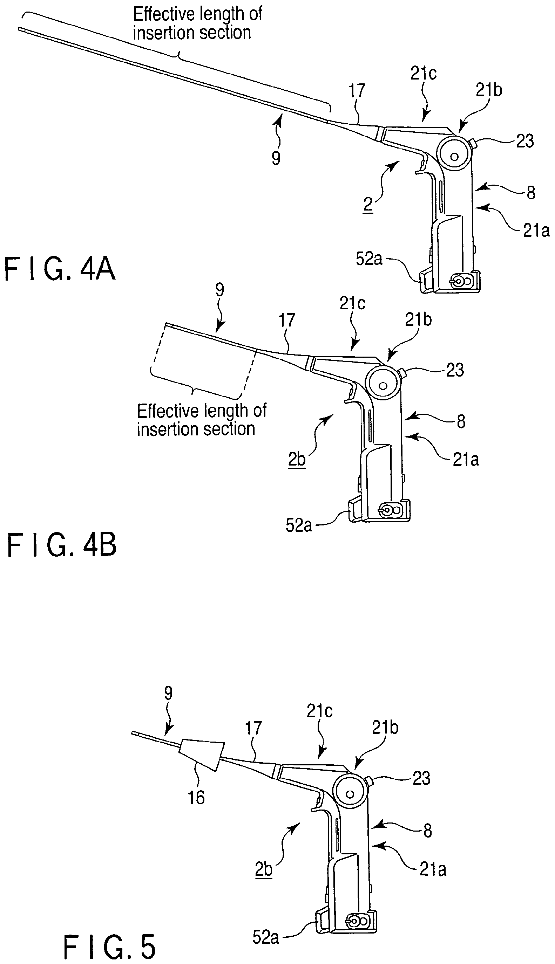

When the endoscope 2 is an endoscope suitable for the otorhinolaryngology in particular, the insertion section 9 of such an otorhinolaryngologic endoscope 2 is nasally inserted, for example. Therefore, the flexible tube section 12 of the insertion section 9 shown in FIG. 4A is flexible and often has a specification that its effective length is approximately 300 mm to be suitable for observation of the hypopharynx or the pharynx.

As shown in FIG. 4B, when such an insertion section 9 is of a short type, since a nasal cavity length of a male adult is usually approximately 60 mm, an effective length of the insertion section 9 of an endoscope 2b is assumed to be approximately 50 to 150 mm so that a region that is observed in the otorhinolaryngology can be covered, or more preferably 50 to 100 mm while considering the nasal cavity length or an allowance length for improvement in handing by an operator (in this case, a length of a bending section occupied in the insertion section 9 is assumed to be approximately 30% to 50%).

Furthermore, when the insertion section 9 of the otorhinolaryngologic endoscope 2 is shortened to, e.g., approximately 50 mm or below, an operator can operate the endoscope 2 by one hand and handle a surgical instrument (not shown) by the other hand.

That is, as denoted by reference number 2b in FIG. 4B, even if the effective length of the insertion section 9 is shortened and the insertion section 9 is set to be substantially vertical to the endoscope operation section 8, the endoscope may be provided as the short-type otorhinolaryngologic endoscope having the flexibility so that the insertion section 9 cannot droop down.

As shown in FIG. 5, a detachable attachment 16 having a diameter larger than a natural orifice such as a nose or an ear may be disposed to the insertion section 9 on the side close to the endoscope operation section 8 to prevent the insertion section 9 from being inserted into an affected area (body cavity) too much. Then, it is possible to regulate insertion of a part of the insertion section 9 close to the rear end side away from the part having the attachment 16 disposed thereto into the body cavity.

It is to be noted that an imaging element (observation optical system) 14 such as a CCD or a CMOS is arranged in the distal end hard section 10 to acquire a picture of a subject S in the body cavity through an objective optical system (observation optical system) 13.

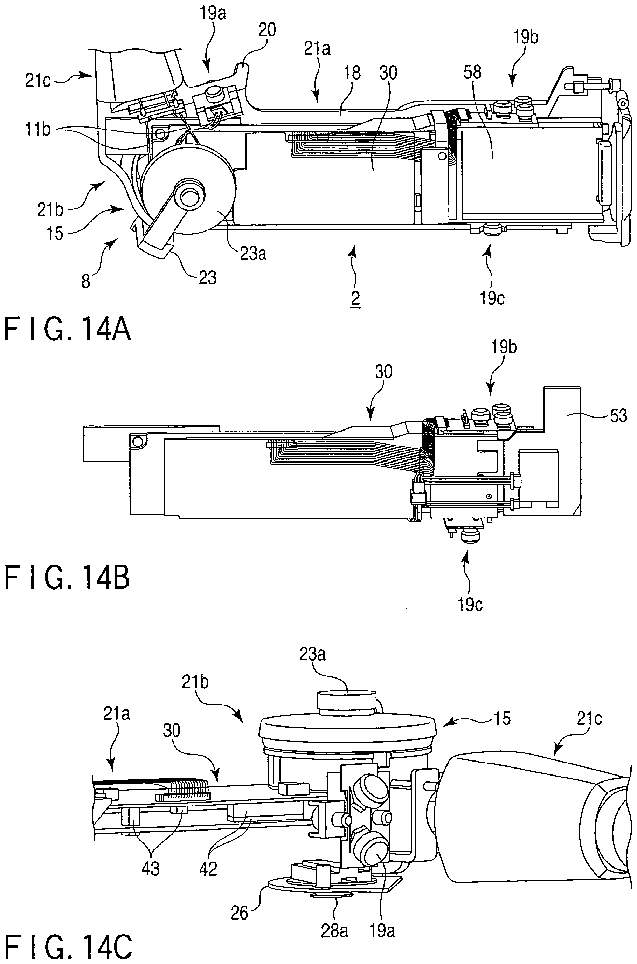

Moreover, the bending section 11 includes a bending tube 11a in which a pair of operation wires 11b (see FIG. 14A) are arranged, and the bending section 11 can bend in two directions (direction U and direction D). Needless to say, the bending section 11 may be configured to bend in four directions.

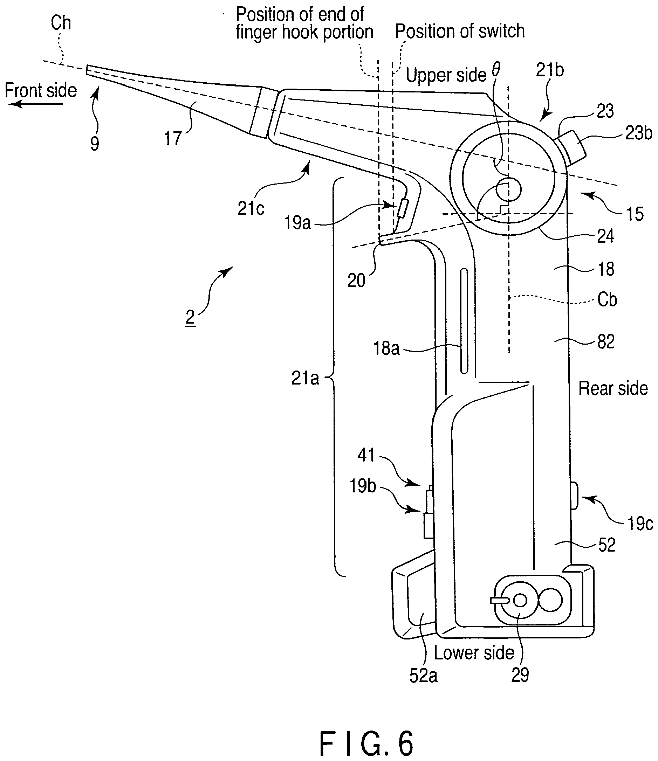

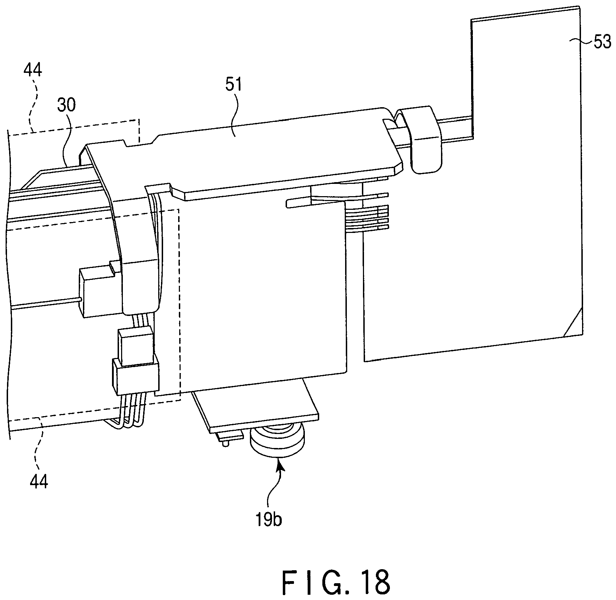

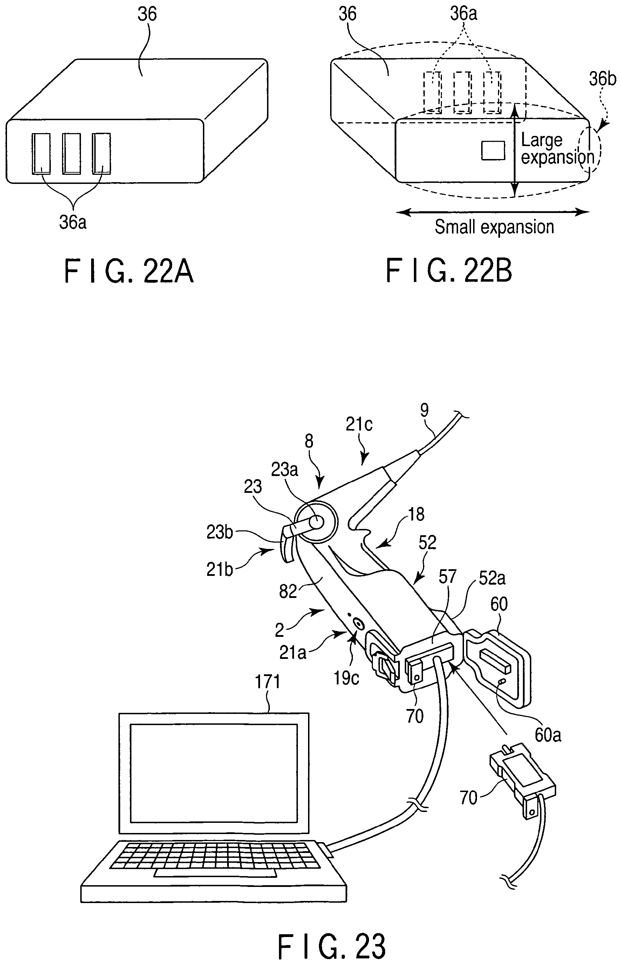

As shown in FIG. 6, the endoscope operation section 8 includes a base section 21a having a gripping section 18 that is extended in the vertical direction and gripped by an operator, a curved section 21b, and a head section 21c to which the rear end portion of the flexible tube section 12 of the insertion section 9 is disposed through the protection hood 17. An outer envelope of the endoscope operation section 8 is formed by disposing a housing 82 made of, e.g., a hard plastic material and a cylindrical body 52 which is also formed of a hard plastic material and arranged in a lower part of the base section 21a. As shown in FIGS. 18A, B and FIG. 22A, the housing 82 forms the outer envelope of a part of the base section 21a (upper part of base section 21a) having the gripping section 18, the curved section 21b, and the head section 21c. An antenna accommodation section 52a of the cylindrical body 52 of the base section 21 and the head section 21c are formed of an electromagnetic wave permeable material in particular. A central axis (longitudinal direction) Ch of the head section 21c extends in a direction having a predetermined angle θ by the curved section 21b with respect to a central axis (longitudinal direction) Cb of the base section 21a, and the endoscope operation section 8 is formed into a substantially-L-like gun type (substantially pistol type) shape. That is, the curved section 21b is arranged between the base section 21a and the head section 21c and sets the head section 21c to face an appropriate aspect in the front-and-back direction with respect to the base section 21a that is long in the vertical direction. The insertion section 9 is extended in a direction that coincides with the central axis Ch of the head section 21c from a distal end of the protection hood 17. Here, the predetermined angle θ is a substantially right angle with respect to the insertion section 9. Incidentally, in case of using the endoscope for, e.g., an otologic application, when inserting the insertion section 9 into a body cavity of a patient who is seated, it is preferable to set the angle θ of the base section 21a to fall within the range of a substantially right angle (90°) to 105° with respect to the insertion section 9 so that an operator can easily grip the base section 21a without bending the flexible tube section 12 of the insertion section 9.

Therefore, the insertion section 9 having the coupled rear end portion can be extended in the front-and-back direction when an operator grips the gripping section 18 of the operation section 8, namely, the insertion section 9 can be extended toward the a patient without straining a wrist of the operator. Therefore, the operator can easily use the endoscope 2, unnatural operations of the endoscope 2 can be reduced for a patient, and hence the patient can comfortably have observation, a treatment, and others using the endoscope 2.

Here, the endoscope 2 is assumed to be held by the operator by one hand as shown in FIG. 7, the vertical direction in FIG. 7 is determined as the vertical direction of the endoscope 2, the upper side of the endoscope operation section 8 is determined as the upper side of the endoscope 2, the lower side of the endoscope operation section 8 is determined as the lower side of the endoscope 2, the left-hand side in FIG. 7 which is the extending direction of the insertion section 9 is determined as the front side of the endoscope 2, the right-hand side in FIG. 7 is determined as the back side of the endoscope 2, the front side in FIG. 7 is determined as the left-hand side of the endoscope 2, and the inner side of FIG. 7 is determined as the right-hand side of the endoscope 2. Moreover, it is preferable for the endoscope 2 to have a substantially horizontally symmetrical shape, and the endoscope 2 may be held by not only the right hand shown in FIG. 7 but also the left hand. Although a bending operation lever 23 that is not horizontally symmetrical is supported on the right-hand side of the endoscope 2 through a supporting point 23a, it may be configured to be supported on the left-hand side through the supporting point 23a.

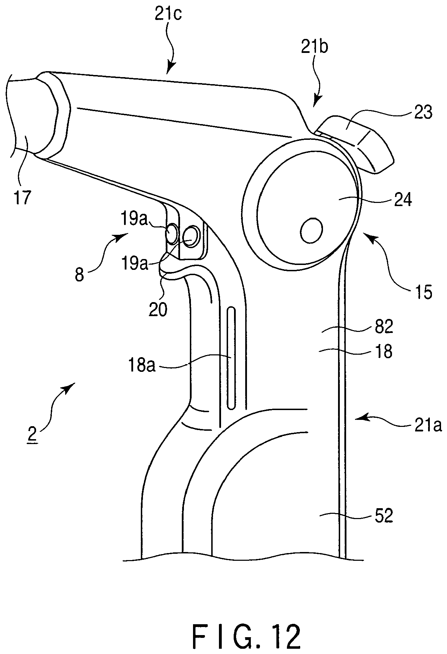

As shown in FIG. 12, a pair of first operation switches 19a are arranged and a finger hook portion 20 abutting on an upper surface of a third finger F3 is formed on a front part of the boundary portion of an upper end portion of the base section 21a of the endoscope operation section 8, the curved section 21b, and the proximal end portion of the head section 21c. The finger hook portion 20 is formed immediately below the first operation switches 19a on the side close to the later-described gripping section 18 rather than the protection hood 17. As shown in FIG. 6, a basal part of the finger hook portion 20 describes a substantially semicircular shape and extends in a direction forming an angle of 90° or above with respect to the longitudinal direction of the gripping section 18 (central part Cb of base section 21a), and an end of the finger hook portion 20 is formed to bulge (protrude) toward the front side beyond the foremost part of each first operation switch 19a. The finger hook portion 20 improves the easiness of gripping and that of pushing of first operation switches 19a and, for example, the third finger F3 can be caught on the portion to prevent the endoscope 2 from falling from an operator's hand when the operator moves his/her hand from the gripping section 18. When the finger hook portion 20 is formed in this manner, the operator can stably hold the endoscope operation section 8 of the endoscope 2 having the gun type shape, and operations of the first operation switches 19a by a second finger F2 can be facilitated. Additionally, since the first operation switches 19a are partitioned with respect to the gripping section 18 by the finger hook portion 20, touching the first operation switches 19a when gripping the gripping section 18 can be avoided.

It is to be noted that the finger hook portion 20 may have an annular shape like a part in which a trigger of a real gun is arranged (trigger guard). At this time, the first operation switches 19a correspond to the trigger of the gun. In this case, even if the operator moves his/her hand from the gripping portion 18, since the annular member is caught by the second finger F2, the endoscope 2 can hardly fall from the operator's hand.

As shown in FIG. 3 and FIG. 7, the base section 21a includes the gripping section 18 gripped by a user (operator) of the endoscope 2 and the cylindrical body 52 cooperating with the gripping section 18 to accommodate the substrate unit 30 or a battery 36 on the outer peripheral surface of the base section 21a. It is preferable for the gripping section 18 to be formed especially on the upper end portion side between the upper end portion and the proximal end portion of the base section 21a. The gripping section 18 is appropriately formed so that it can be easily gripped and, in this example, as shown in FIG. 7, the gripping section 18 is held in a state in which a first finger F1 is arranged in a concave portion 23b of the bending operation lever 23, the second finger F2 is arranged on the first operation switch 19a, the third finger F3 is arranged on the lower side of the finger hook portion 20, and a fourth finger F4 and a fifth finger F5 are arranged on the front side of the gripping section 18 of the base section 21a. Here, with regard to an outer shape (outer peripheral length) of the gripping section 18, the outer shape (outer peripheral length) is formed to be smaller than the cylindrical body 52 in accordance with size of hand. Further, it is preferable for a region of the gripping section 18 in the vertical direction to be formed so that the third finger F3, the fourth finger F4, and the fifth finger F5 can be fitted in the region when the gripping section 18 is held as shown in FIG. 7.

It is to be noted that, as shown in FIG. 6 and FIG. 12, a rib (protrusion) 18a that is long in the vertical direction and exercises an antislip function for the operator's hand is formed on each of a left front part and a right front part. Furthermore, although not shown, bases and their surrounding areas of fingers F2, F3, F4, and F5 come into contact with the ribs 18a, and finger pads at fingertips or finger pads between first joints and second joints of the third finger F3, the fourth finger F4, and the fifth finger F5 are caught on the ribs 18a as shown in FIG. 7.

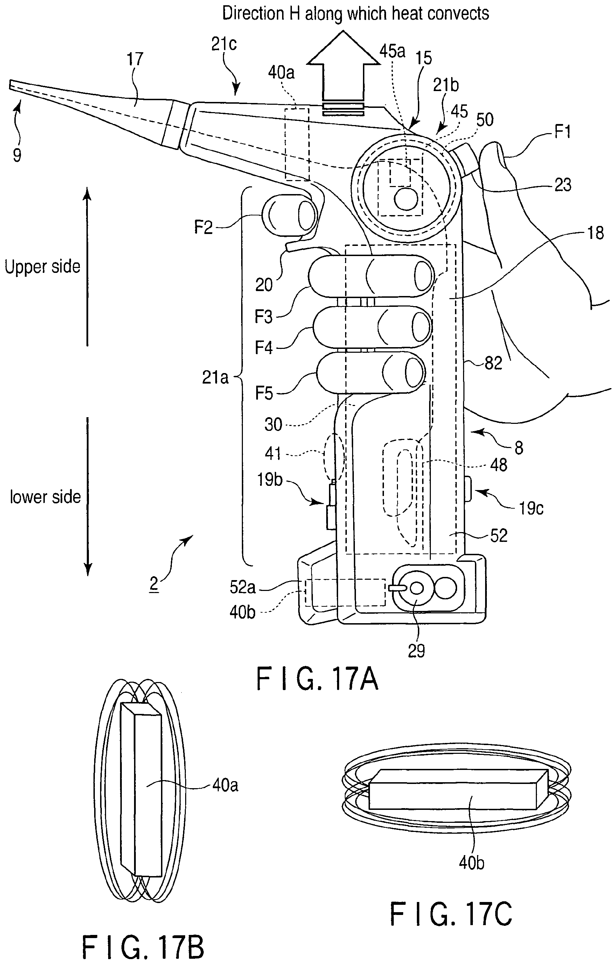

As depicted in FIG. 3 and FIG. 17A, the substrate unit 30 is arranged in the base section 21a. The base unit 30 and the later-described battery 36 are accommodated in the cylindrical body 52 of the base section 21a. Furthermore, the second and third operation switches 19b and 19c arranged on the substrate unit 30 is provided on the cylindrical body 52. In this embodiment, the second operation switch 19b is provided on the front side portion of the cylindrical body 52, and the third operation switch 19c is provided on the rear side portion of the cylindrical body 52, but arranging these switches 19b and 19c on the left side portion or the right side portion of the cylindrical body 52 is also preferable.

As shown in FIG. 6, a vent mouth ring 29 communicating with the inside of the endoscope 2 is provided to protrude at a position near the lower end portion of the cylindrical body 52 of the base section 21a (position near left lower end portion in this embodiment). At the time of a water tightness test of the endoscope 2, an adapter (not shown) provided to an external air supply device is connected to the vent mouth ring 29, whereby pressurized air is supplied to the inside of the endoscope 2 through the vent mouth ring 29.

The vent mouth ring 29 also functions as a pressure regulation valve for the inside of the endoscope 2 at the time of a sterilization process of the endoscope 2 using, e.g., high-pressure moisture vapor.

Furthermore, as shown in FIG. 17A, the antenna accommodation section 52a accommodating the second antenna 40b protrudes on the front side of the lowermost portion of the cylindrical body 52. In this embodiment, the antenna accommodation section 52a protrudes on the foremost side of the base section 21a (side very close to rear end portion of insertion section 9). The antenna accommodation section 52a is formed of a material through which electromagnetic waves can be transmitted (electromagnetic wave permeable material), e.g., a plastic material. Incidentally, it is preferable for the outer peripheral surface of the cylindrical body 52 to be formed of the same material as the antenna accommodation section 52a, and portions other than the antenna accommodation section 52a may be formed of, e.g., a metal material through which electromagnetic waves are hardly transmitted.

A bending operation section (bending operation mechanism) 15 that is operated to bend the bending section 11 of the insertion section 9 is arranged at the curved section 21b arranged at the upper end portion of the base section 21a. The bending operation section 15 includes a bending operation lever 23 that can rotate in a predetermined range with a supporting point 23a shown in FIG. 14A and FIG. 14C at the center. It is to be noted that the bending operation lever 23 extends from the supporting point 23a on the right side surface toward the rear upper side of the endoscope operation section 8 and also extends in the horizontal direction on the rear upper side. A concave portion 23b configured to arrange the finger pad of the first finger F1 at a predetermined position is formed at an end portion of the bending operation section 15 distant from the supporting point 23a. Therefore, the bending operation lever 23 in the bending operation section 15 is provided outside of the endoscope operation section 8, and it can be operated by the first finger F1 of the hand.

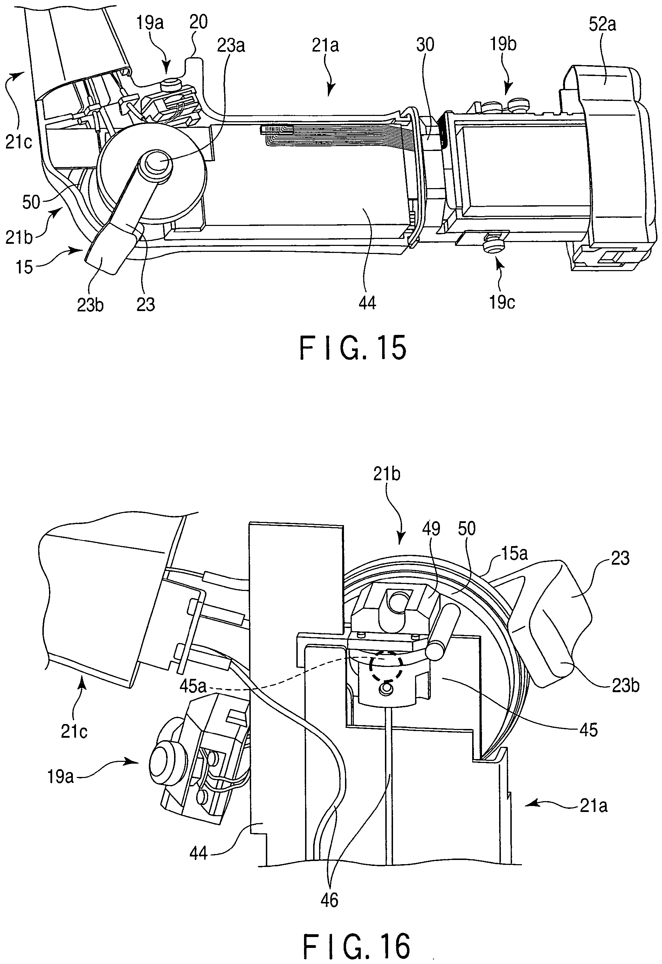

A drum 15a (see FIG. 16) connected to the supporting point 23a of the bending operation lever 23 (see FIG. 16) is arranged in the curved section 21b of the endoscope operation section 8. Therefore, when the bending operation lever 23 is operated to rotate, the drum rotates in accordance with the rotational motion. Since the operation wires 11b are wound around the drum 15a, rotating the bending operation lever 23 enables moving forward or backward the operation wires 11b along its axial direction. Therefore, the bending tube 11a of the bending section 11 can be bent. That is, the bending operation lever 23 is arranged as a rotating member configured to bend the bending section 11 by moving forward or backward the operation wires 11b depicted in FIG. 14A.

Furthermore, it is preferable to determine a position of the concave portion 23b of the bending operation lever 23 shown in FIG. 15 and FIG. 16 which is substantially symmetrical with respect to the first switch 19a with the supporting point 23a at the center as a position at which the bending section 11 is straightened. In this case, arranging the first finger F1 in the concave portion 23b of the bending operation lever 23 enables supporting force in a pushing direction when pushing the first operation switches 19a. Therefore, an operator can readily operate the first operation switch 19a and also operate the bending operation lever 23 to rotate while stably gripping the gripping section 18.

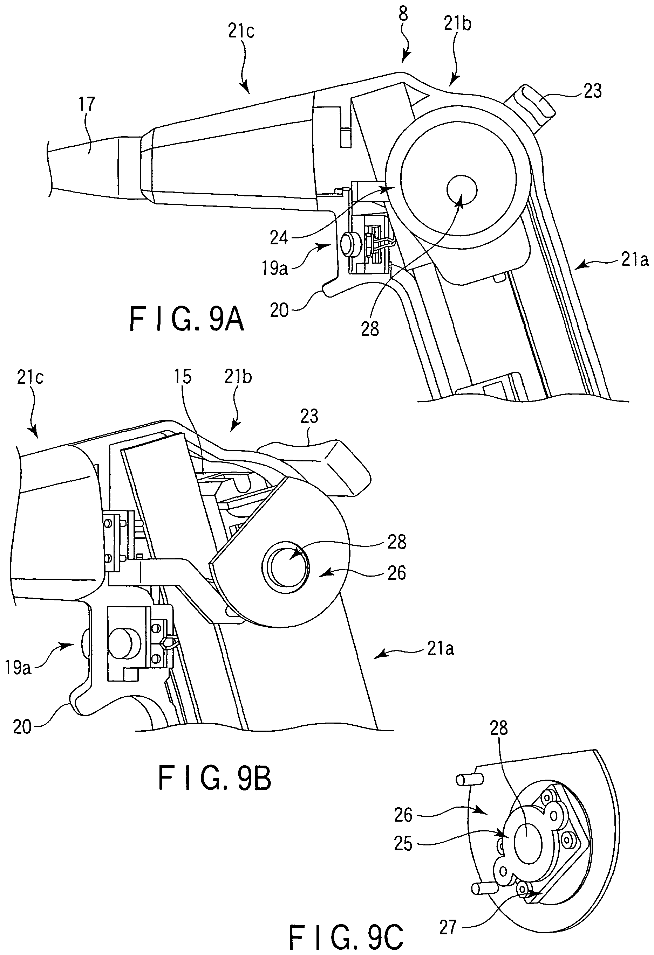

As shown in FIG. 9, an outer sheath cover 24 is arranged on an outer sheath portion accommodating the bending operation section 15 in the curved section 21b.

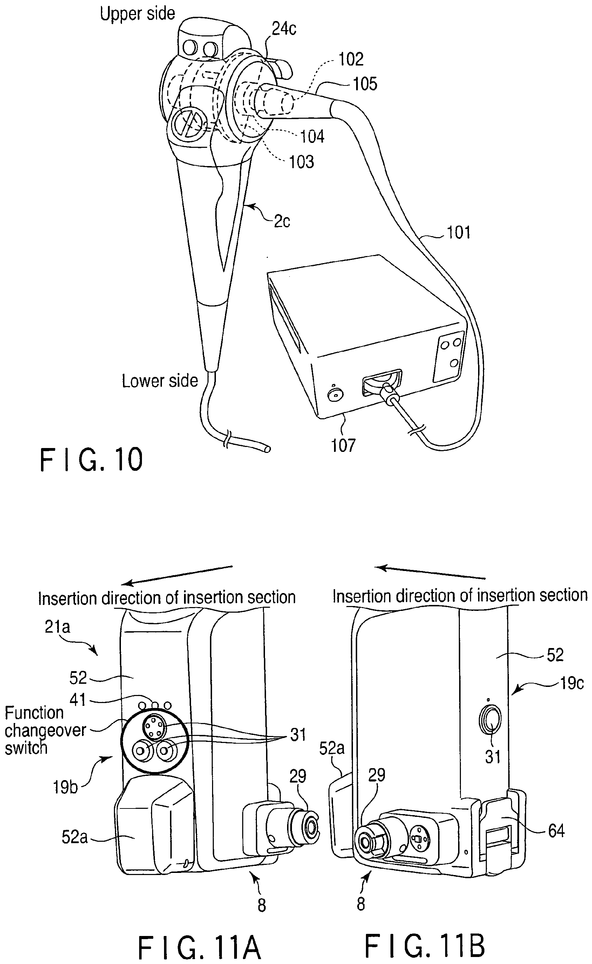

Meanwhile, in a conventional endoscope 2c whose vertical direction is specified as shown in FIG. 10, a cable 101 connecting a main body of the endoscope 2c to a processing device 107 extends from an outer sheath cover denoted by reference number 24c, and the cable 101 is directly fixed to a metal fixing member 104 provided on an inner frame 103 by a metal mouth ring 102. A protection hood 105 configured to avoid flexion of the cable 101 is provided at a connecting portion between the metal component 102 of the cable and the fixing member 104, thereby maintaining an insulated state.

The endoscope 2 according to this embodiment is the endoscope adopting a wireless communication system, and the endoscope operation section 8 is not connected to the processing device 3 through the cable 101 as different from the conventional endoscope 2c. Therefore, when the outer sheath cover 24 is fixed by the metal mouth ring 102, the fixing member 104, or the protection hood 105, the component which fixes the outer sheath cover 24 greatly protrudes, which is an obstacle when an operator operates the endoscope 2.

Furthermore, when the metal component, e.g., the metal mouth ring 102 or the fixing member 104 is utilized to fix the outer sheath cover 24 as it is, the insulation between various kinds of electronic circuits provided in the endoscope operation section 8 and an outer sheath metal cannot be realized. When the various electronic circuits in the endoscope operation section 8 cannot be insulated from the outer sheath metal as described above, electric safety problems such as an influence of static charge or noise occur.

Thus, in the endoscope 2 according to this embodiment, as shown in FIG. 9B and FIG. 9C, a screw 28 of the outer sheath cover 24 is screwed and fixed to an outer sheath cover fixing member 25 to sandwich the outer sheath cover 24 between the outer sheath cover fixing member 25 which does not protrude from the outer sheath cover 24 in the left direction in this embodiment and a frame 26 which is a part of a framework provided in the endoscope 2 through an insulating member 27, thereby maintaining the insulated state between the frame 26 electrically conductive with respect to the various electronic components and connected as a ground and the outside.

As shown in FIG. 17A, a light source unit 45 is arranged on the left side in the housing 82 of the base section 21a, the curved section 21b, or the head section 21c of the endoscope 2 according to this embodiment through a bending operation section main body 50 which is a metal member of the bending operation section 15 and a light source attachment section 49 at the upper end portion of the substrate unit 30. That is, the substrate unit 30 and the bending operation section 15 are connected in a predetermined state. The light source unit 45 includes an illumination light source 45a such as an a light-emitting diode (LED) and utilizes electric power supplied from the battery 36 to cause light emission from the illumination light source 45a. Illumination light generated by the light emission from the illumination light source 45a enables light to exit from an illumination lens 47 of the distal end hard section 10 via the proximal end to the distal end of a light guide 46 internally inserted in the head section 21c and the insertion section 9, thereby illuminating a subject S.

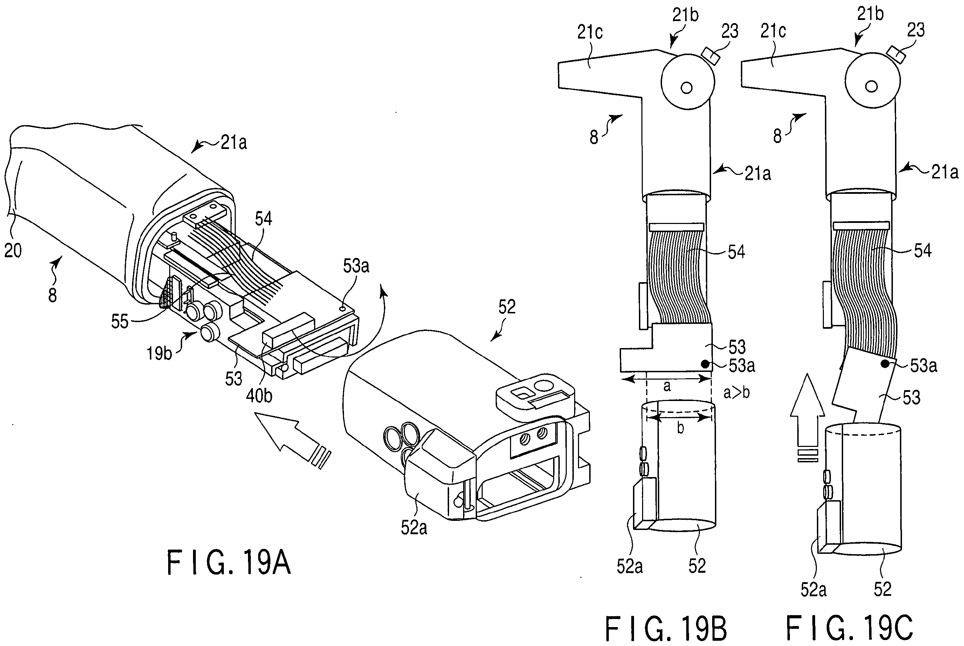

As shown in FIG. 19A, the light source unit 45 and the light source attachment section 49 of the gun type endoscope 2 are arranged on the upper side in the operation section 8 to prevent heat from the light source 45a from being conducted to electronic components implemented on the substrate unit 30. Such a configuration can be likewise applied to the conventional endoscope 2c depicted in FIG. 10.

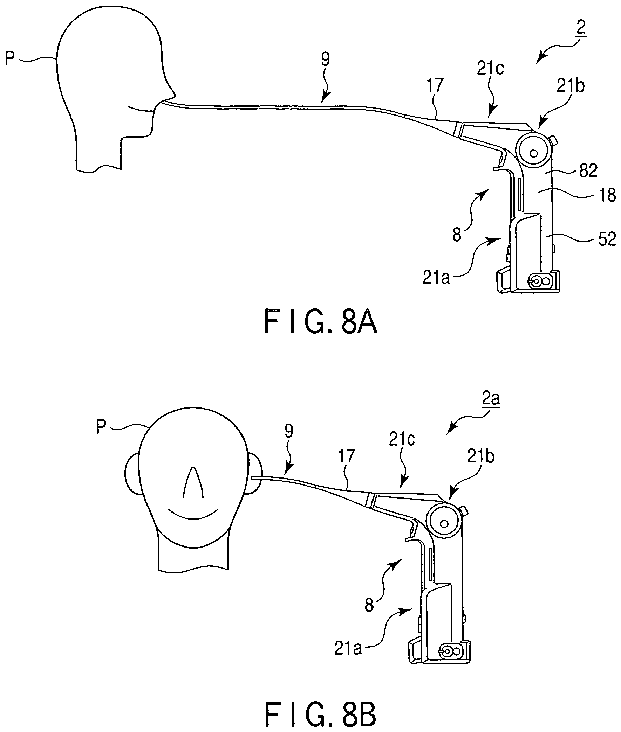

When the endoscope 2 according to this embodiment has the above-described gun type shape, the endoscope operation section 8, as shown FIG. 8A and FIG. 8B, can be easily used and effective when an operator faces a patient P to insert the insertion section 9 into a body cavity through the nose or the ear and an examination or a treatment is thereby conducted.

The first to third operation switches 19a, 19b, and 19c will now be described.

As shown in FIG. 2, the first to third operation switches (push buttons and others) 19a, 19b and 19c which perform various kinds of settings or remotely operate the processing device 3 are arranged on the endoscope operation section 8. The pair of first operation switches 19a are arranged on the front side part of the boundary between the upper end portion of the base section 21a, the curved section 21b, and the proximal end portion of the head section 21c of the endoscope operation section 8, and they are operated by, e.g., the second finger F2. The second and third operation switches 19b and 19c are formed at positions where they do not catch (not come into contact with) the hand when the gripping section 18 of the endoscope 2 is held as shown in FIG. 7.

As shown in FIG. 3, the first to third operation switches 19a, 19b and 19c are connected with the substrate unit 30 in the endoscope main body 8 through respective wiring cables. It is to be noted that, when the first to third operation switches 19a, 19b and 19c are pushed, settings of on/off of a power supply, brightness, white balance, enhancement, channel switching, and others can be performed.

The first to third operation switches 19a, 19b and 19c are set to exercise various functions when being pushed, respectively. With regard to the second operation switches 19b shown in FIG. 11A, shapes (protrusions are provided to key tops 31 and the number of the protrusions is changed, for example), colors (e.g., black, gray, and any other color), and arrangement (surface of the endoscope operation section 8 on which the finger is arranged and its back surface, for example) of key tops 31 to press push buttons by a finger can be changed in accordance with each function or a use frequency, thus enabling identification based on visual confirmation and tactile sensation (e.g., each function and a use frequency).

Moreover, each first operation switch 19a is arranged in such a manner that a direction of pressing the first operation switch 19a is preferably horizontal to the front-and-back direction (longitudinal direction) of the insertion section 9. Therefore, the endoscope operation section 8 can be prevented from being displaced in, e.g., the vertical direction due to impetus involved by a pressing operation performed when pressing each first operation switch 19a during use of the endoscope 2. Additionally, as described above, since the second finger F2 can be utilized to push down the first operation switch 19a in a state in which the first finger F1 is placed in the concave portion 23b of the bending operation lever 23 when the first operation switch 19a is pressed, the pressing operation for the first operation switch 19a can be easily performed.

As shown in FIG. 12, providing the plurality of key tops 31 to the function switching first operation switches 19a is preferable, and the two key tops 31 are provided in parallel in this embodiment. In this case, these key tops 31 do not face the front side (direct front) but they face a slightly inclined direction. One of the two key tops 31 (e.g., the left key top 31) is pressed by, e.g., the finger pad at the tip of the second finger F2, and the other (e.g., the right key top 31) is pressed by the finger pad between the first joint and the second joint or the finger pad between the second joint and the root of the second finger F2. Therefore, the two key tops 31 are provided at different angles so that the key tops 31 can be pressed in a direction substantially orthogonal to a direction in which the operator's second finger F2 is crooked in accordance with each joint of the second finger F2. That is, in this embodiment, the two first operation switches 19a are provided in parallel, and the pressing direction of each key top 31 faces the direction substantially orthogonal to the finger pad of the second finger F2. Since the key tops 31 face the different directions, the first operation switches 19a can be easily separately pressed to prevent the plurality of arranged first operation switches 19a from being erroneously pressed.

It is to be noted that, when the first operation switches 19a have two key tops 31, inclining the two key tops 31 with respect to the front side (front) at equal angles is preferable. If so, even if the endoscope 2 according to this embodiment can be used without discomfort irrespective of the left hand or the right hand that holds the second endoscope 2.

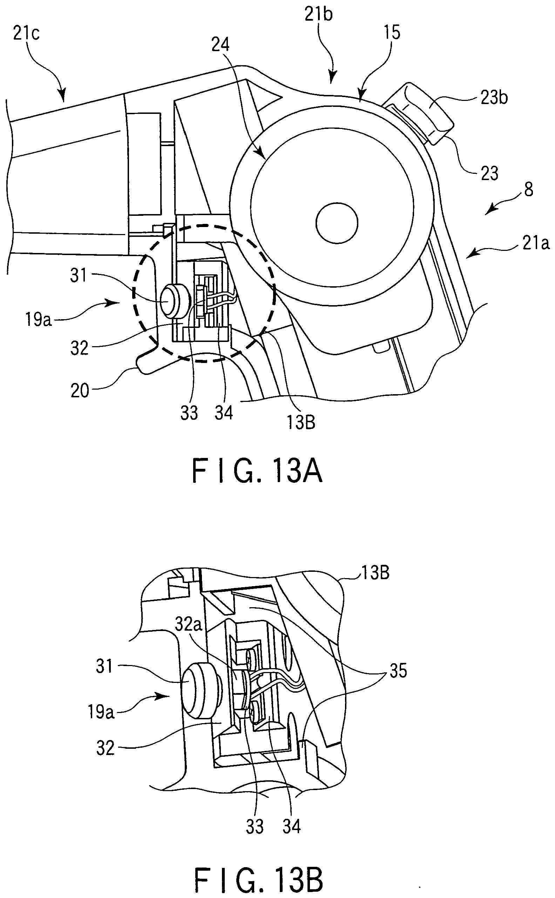

As shown in FIG. 13A and FIG. 13B, when each first operation switch 19a is constituted of a push button, the first operation switch 19a includes the key top 31 pressed by an operator, a switch portion 33 such as a tact switch which operates by pushing the key top 31 and which performs, e.g., circuit switching, a key top fixing member 32 to fix the key top 31, and a switch fixing member 34 to fix the switch portion 33.

When the key top 31 is clamped by a nut 32a through the key top fixing member 32, it is fixed to the endoscope operation section 8. The switch portion 33 is positioned with respect to the switch fixing member 34 and then fixed through, e.g., a screw. Further, the key top fixing member 32 and the switch fixing member 34 are disposed to the endoscope operation section 8 via, e.g., a screw with the same plane of an attachment guide 35 provided to the endoscope operation section 8 being determined as a reference. Therefore, at the time of assembling the endoscope operation section 8, the switch portion 33 can be simply and accurately positioned with respect to the key top 31.

The substrate unit 30 included in the endoscope operation section 8 will now be described.

As shown in FIG. 3, the substrate unit 30 is a set of electronic circuit substrate included in the base section 21a of the endoscope operation section 8. The substrate unit 30 includes a switch circuit 37 which is accommodated in the lower end portion of the base section 21a of the endoscope 2 and switches power from the battery 36, e.g., a lithium ion rechargeable battery serving as a drive power supply of the endoscope 2 to each circuit in accordance with operation signals and others from the first to third operation switches 19a, 19b, and 19c, an image processing circuit 38 which executes processing, e.g., compression to a picture signal of the inside of a body cavity acquired by the imaging element 14, a wireless circuit 39 which converts the picture signal into a wireless signal, and first and second antennas 40a and 40b to transmit the wireless signal to reception antennas 7 attached to the external processing device 3.

In the endoscopic system 1, to avoid wireless cross talk, a plurality of wireless channels can be selected. Therefore, the substrate unit 30 has a wireless channel switching function that enables selecting and changing over the wireless channels by using, e.g., the second operation switches 19b. The substrate unit 30 has non-illustrated channel setting storing means to enable holding a selected channel setting even if the power supply of the endoscope 2 is turned off or supply of the power from the battery 36 is interrupted.

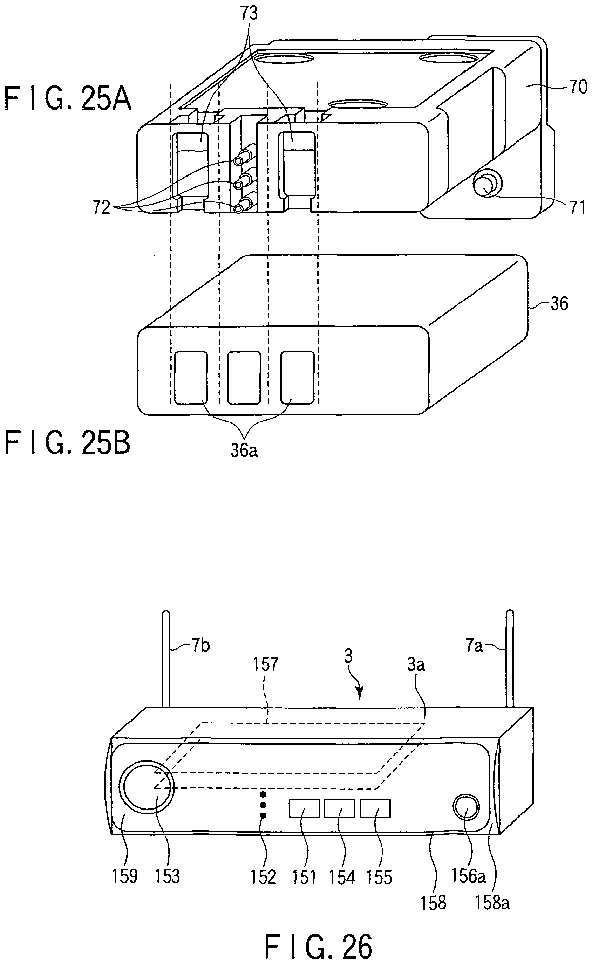

It is to be noted that, since the wireless signal can be transmitted/received only between the endoscope 2 and the processing device 3 having the same channel setting, a wireless channel changeover switch 151 that enables the same channel setting as that of the endoscope 2 is also provided to the processing device 3 depicted in FIG. 26. A channel set in both the endoscope 2 and the processing deice 3 is displayed in an indication lamp 41 such as an LED provided at, e.g., a position adjacent to the second operation switches 19b (upper side in this embodiment) and a channel display section 152 of the processing device 3 shown in FIG. 26, respectively.

It is to be noted that, the three second operation switches 19b arranged on the base section 21a of the endoscope 2 may be arranged to be placed at vertexes of a triangle here as shown in FIG. 11A, but three switches may be provided in parallel, for example. Further, the channels are switched based on the number of times of pressing the second operation switches 19b. Then, the two remaining switches in the second operation switches 19b can be used for, e.g., setting of other functions.

Furthermore, different channels may be selected and set when these second operation switches 19b are pressed.

Moreover, the third operation switch 19c functions as a power supply switch, and the electric system of the endoscope 2 can be appropriately terminated by pressing the third operation switch 19c for several seconds. On the other hand, since the electric system is not terminated when the third operation switch 19c is pressed for a short time, erroneous operations or malfunctions of the third operation switch 19c can be avoided.

As shown in FIG. 14B, in the substrate unit 30, the switch circuit 37, the image processing circuit 38, and the wireless circuit 39 that are various electronic circuits for executing electricity related processing (pictures, wireless, the antennas, the power supply) of the endoscope 2 or the electronic substrates having the various electronic components implemented thereon are electrically connected by, e.g., an inter-substrate connector 42 implemented on the substrate, thereby assembling one unit.

Usually, when examining such electronic circuits, it is often the case that the respective substrates are connected in accordance with a change in operation state in an assembly process or examination jig and others are utilized to gradually perform the examination. The substrate unit 30 has a configuration that all the respective electronic substrates having the main function are connected to be formed as one unit. Therefore, the control can advance to the examination process in a state in which the respective substrates are integrally connected in the assembly process of the endoscope 2. Additionally, the control can advance to the next assembly process (process of assembling substrate unit 30 in endoscope operation section 8) without change from the state in which the examination process is terminated, thereby improving assembly efficiency.

When implementation displacement of the inter-substrate connector 42 is significant, a load may be possibly applied to each fixed substrate. Thus, as shown in FIG. 14C, when spacers 43 are sandwiched between the respective substrates and both sides are fixed by screws, the inter-substrate connector 42 is connected without applying a load on the respective substrate or the inter-substrate connector 42.

When laminating and arranging the respective substrates, as shown in FIG. 15, an inner frame 44 which is long in the vertical direction and formed of, e.g., a metal is provided in parallel to each substrate surface of the substrate unit 30. As a result, the components included in the endoscope operation section 8 can be integrated as a structure in which the inner frame 44 is combined with the substrate unit 30. Therefore, the number of the components can be reduced, and the assembly efficiency can be further improved. At the same time, heat generated from the electronic components can be efficiently released to other portions through the inner frame 44 having high exoergic properties.

It is to be noted that, to efficiently transfer the heat from the electronic components to the inner frame 44, non-illustrated heat transferring means such as an electrothermal sheet or a gel sheet may be sandwiched and disposed between each substrate and the inner frame 44.

A subject image of the subject S illuminated with the illumination light from the light source 45a of the light source unit 45 is formed on the imaging element 14 by the objective optical system 13 included in the distal end hard section 10 and acquired by the imaging element 14. The imaging element 14 is connected to the image processing circuit 38 in the substrate unit 30 provided in the base section 21a through an imaging cable (observation optical system) 48. Therefore, an imaging signal acquired by the imaging element 14 is output via the imaging cable 48 to the image processing circuit 38 where various kinds of image processing are executed. A picture signal is output from the image processing circuit 38 to the wireless circuit 39, and it is converted into a wireless signal by the wireless circuit 39. The wireless signal is output from the wireless circuit 39 to the first and second transmission antennas 40a and 40b, and it is transmitted from the first and second transmission antennas 40a and 40b to the processing device 3.

As described above, the light source unit 45 is coupled with a bending operation section main body 50 which is a metal member of the bending operation section 15 by the light source attachment section 49 placed above the substrate unit 30.

An electronic component which is weak with respect to an increase in temperature may be implemented on each substrate in the substrate unit 30. Therefore, as shown in FIG. 17A, considering a situation that heat H from the light source unit 45 convects to a relatively upper side, the light source 45a which is a component that generates heat the most is arranged on the upper side (side close to bending operation section 15 rather than gripping section 18) when operator holds the endoscope 2, and the substrate on which each electronic component is implemented is arranged to be placed on the lower side than the light source unit 45 when the operator grips the endoscope 2, respectively. That is, since warm air has characteristics of moving to the upper side, the electronic components are configured to be hardly affected by heat from the light source 45a.

Further, the imaging cable 48 is arranged along the inner frame 44 and connected to the inner frame 44. Here, the inner frame 44 is a comprehensive ground for the respective electronic components. Therefore, noise from the imaging cable 48 that affects EMC can be absorbed into the inner frame 44. That is, the noise from the imaging cable 48 through which a signal from the imaging element 14 is transmitted can be absorbed into the inner frame 44.

Furthermore, the substrate unit 30 has improved assembly properties when assembling (attaching) in the endoscope operation section 8 of the endoscope 2. As shown in FIG. 18, although the substrate unit 30 is configured to be disposed in the endoscope operation section 8 while being covered with the inner frame 44, the substrate 30 cannot be surrounded by, e.g., a case in the assembly process. Therefore, a reinforcing bracket 51 that connects each inner frame 44 that fixes the substrate is provided, thereby avoiding occurrence of deformation or a short circuit (decrease in insulation) of each substrate when the inner frame 44 fixing opposed substrates are deformed. The bracket 51 also functions as a pedestal that supports components constituting the second and third operation switches 19b and 19c and also has an effect of improving the exoergic properties of the substrate unit 30.

As shown in FIG. 19A, when assembling the endoscope operation section 8, the substrate unit 30 of the endoscope operation section 8 is inserted into the cylindrical body 52 which is a part of the base section 21a. At this time, as shown in FIG. 19B, a width of the lowermost portion of the substrate unit 30 is larger than an inner dimension of the cylindrical body 52, and the substrate unit 30 cannot be inserted as it is. Thus, in an electronic substrate at the lower end portion of the substrate unit 30, a part larger than the inner width of the cylindrical body 52 is constituted by combining a rigid substrate 53 to a flexible substrate 54 that can rotate with respect to the rigid substrate 53 with a predetermined part being used as an axis 53a. Therefore, when fitting the cylindrical body 52 to the substrate unit 30, as shown in FIG. 19C, interference is prevented from occurring between the cylindrical body 52 and the rigid substrate 53 by rotating the rigid substrate 53 on the axis 53a with respect to the flexible substrate 54. It is to be noted that, after fitting the cylindrical body 52, the rigid substrate 53 is rotated to be restored to the original state with respect to the axis 53a, whereby the second antenna 40a is accommodated in the antenna accommodation section 52a of the cylindrical body 52.

Further, to prevent a wiring line having an end portion of the imaging cable 48 shown in FIG. 17A soldered thereto from being disconnected during the assembly of the endoscope operation section 8, a substrate 55 having the end portion of the imaging cable 48 soldered thereto (see FIG. 19A) is arranged on the outermost surface of the substrate unit 30, and outer sheath members, e.g., the cylindrical body 52 are disposed while performing visual confirmation. Adopting such a configuration enables reducing a possibility that the wiring line having the end portion of the imaging cable 48 soldered thereto is caught by the internal structure of the substrate unit 30 and thereby disconnected, for example.

As shown in FIG. 11, the base section 21a of the endoscope operation section 8 of the endoscope 2 includes on its front side the indication lamp 41 as indicating means such as an LED that indicates a remaining level of the battery 36 or a communication state. In this embodiment, the indication lamp 41 is arranged on the side immediately above the second operation switches 19b. The indication lamp 41 indicates the green color during normal wireless communication with the processing device 3. Furthermore, it emits light or blinks in different patterns in accordance with a situation of the endoscope 2, e.g., emitting green light when a remaining level of the battery 36 is sufficient or yellow light when the remaining level is reduced. Therefore, an operator can easily confirm a wireless communication status of the endoscope, a remaining battery level, and others.

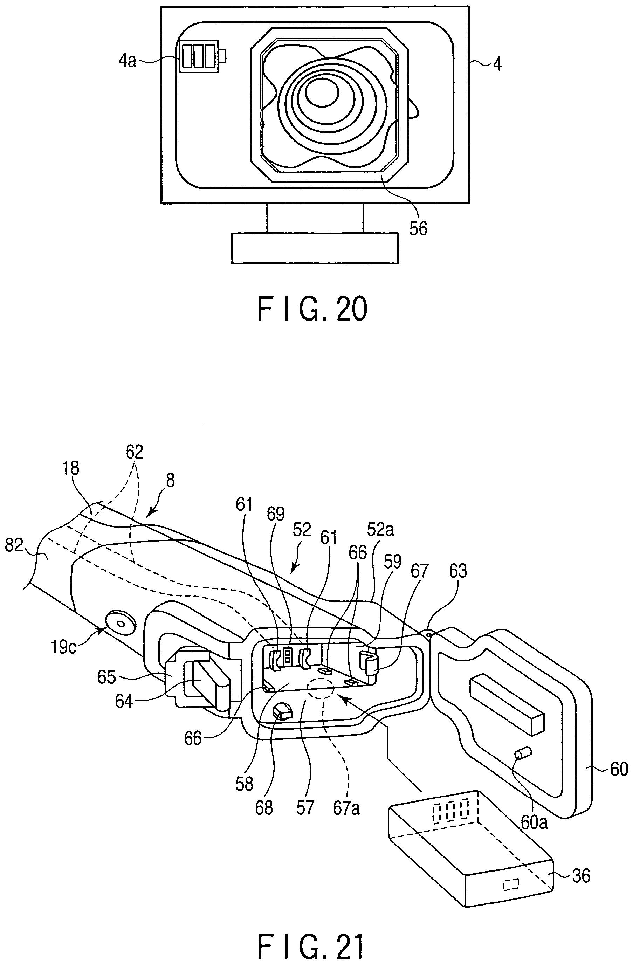

When the remaining level of the battery 36 is low, the endoscope 2 wirelessly transmits to the processing device 3 an electric signal indicating this state, and the processing device 3 shows a state in which the remaining level of the battery 36 in the endoscope 2 is low in a portion denoted by reference number 4a at the upper left of the display device 4 (see FIG. 20). It is to be noted that information concerning the remaining level of the battery 36 displayed in the display device 4 is displayed in detail based on the indication (e.g., changing a color of lighting of the indication lamp 41) of the endoscope operation section 8. Moreover, as another example, the display device 4 may display, e.g., three split signs indicative of the remaining battery levels, all the three split signs may be turned on when the remaining level of the battery 36 is sufficient, and the three signs may be sequentially turned off as the remaining level of the battery 36 is lowered.

It is to be noted that the illumination light source 45a and the indication lamp 41 are arranged at different distant portions to prevent an operator from erroneously recognizing leak light of the illumination light source 45a in the endoscope operation section 8 entering a portion having the indication lamp 41 disposed thereto. Additionally, a non-illustrated light shielding member may be interposed between the illumination light source 45a and the indication lamp 41.

The antennas arranged in the endoscope 2 according to this embodiment will now be described.

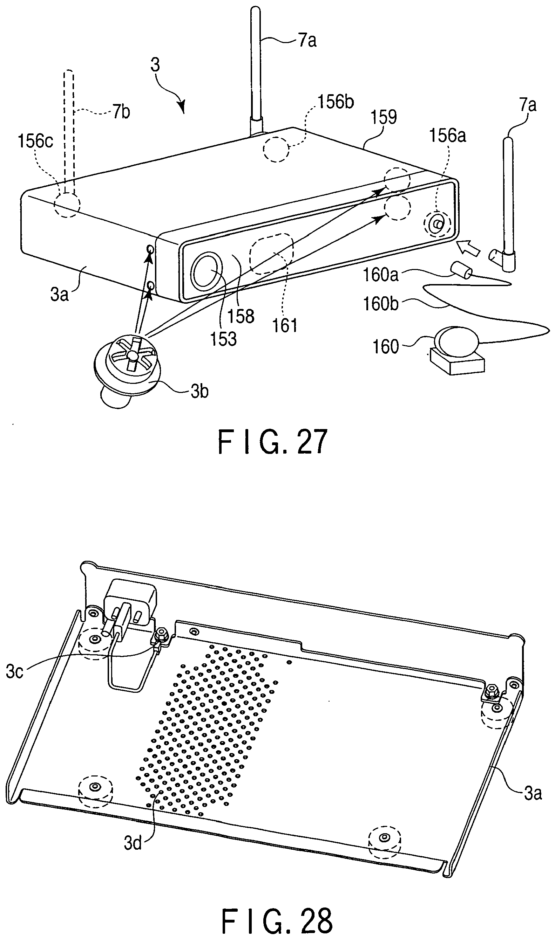

As shown in FIG. 3 and FIG. 17, a wireless communication antenna which is provided to the substrate unit 30 of the endoscope 2 and configured to wirelessly communicate with the external device are formed as, e.g., a diversity antenna including a plurality of antennas. Therefore, since the plurality of antennas (see FIG. 26 and FIG. 27) can be connected to the endoscope 2 or the processing device (external device) 3 to effect reception while switching the antenna with the excellent reception status as needed, an endoscopic image and others can be more assuredly transmitted/received.

The wireless communication antenna in this embodiment includes the first antenna 40a arranged at the upper end portion of the gripping section 18 in the endoscope operation section 8, i.e., a position closer to the insertion section 9 than the griping section 18 and the second antenna 40b arranged at the lower end portion of the base section 21a, i.e., a position farther from the insertion section 9 than the gripping section 18. The first antenna 40a shown in FIG. 17B is arranged in the head section 21c above the first operation switches 19a on which the second finger F2 is arranged as shown in FIG. 17A. That is, the first antenna 40a is provided on the front side apart from a position in the endoscope 2 at which a hand is arranged. At a position in the head section 21c where the first antenna 40a is arranged, a metal body is not provided in later-described orientation directions of electromagnetic waves of the first antenna 40a, thereby facilitating stable transmission of the electromagnetic waves. The second antenna 40b shown in FIG. 17C is arranged in the antenna accommodation section 52a on the front side of the base section 21a at the lowermost portion of the base section 21a sufficiently apart from the right hand as depicted in FIG. 17A.

When at least the two antennas 40a and 40b are arranged at positions in the endoscope operation section (endoscope main body) 8 apart from the gripping section 18, directivities of the antennas 40a and 40b can be prevented from being affected by a human body, and the electromagnetic waves can be assuredly transmitted/received with respect to other devices.

Moreover, the first antenna 40a is arranged at a position closer to the insertion section 9 than the metal frame 44 (electronic component other than the first antenna 40a) in the endoscope operation section 8, and the second antenna 40b is arranged at a position farther from the insertion section 9 than the metal frame 44 4 (electronic component other than the first antenna 40a) in the endoscope operation section 8. In other words, the antennas 40a and 40b are arranged to be apart from the metal frame (metal body) 44. Therefore, when the metal body is distanced from the orientation directions of the electromagnetic waves from the antennas 40a and 40b, the electromagnetic waves from the antennas 40a and 40b can be stably transmitted or received. That is, it is possible to prevent the transmission/reception of the electromagnetic waves using the antennas 40a and 40b from being affected. Therefore, the smooth manipulation of the endoscope 2 that is difficult in conventional examples can be carried out.

The first and second antennas 40a and 40b arranged in the endoscope operation section 8 as shown in FIG. 17A have the directivities like describing a figure eight as depicted in FIG. 17B and FIG. 17C. Additionally, as shown in FIG. 17A, the first antenna 40a and the second antenna 40b are disposed to face different directions (e.g., to be orthogonal to each other). Further, the first antenna 40a is covered with the head section 21c that hardly inhibits the electromagnetic waves in the orientation directions of the electromagnetic waves, and the second antenna 40b is likewise covered with the antenna accommodation section 52a that hardly inhibits the electromagnetic waves in the directions of the electromagnetic waves.

Therefore, the range that the first and second antennas 40a and 40b can assuredly transmit/receive the electromagnetic waves substantially evenly spreads in an examination room, and wireless communication can be stably performed between the first and second antennas 40a and 40b and the reception antennas 7 of the processing device 3 even if the endoscope operation section 8 is moved during use of the endoscope 2.

Here, components constituted of metal parts (metal bodies), e.g., an electric circuit, the inner frame 44 which is a framework of the endoscope operation section 8, and others are intensively provided in the base section 21a of the endoscope operation section 8. For example, the first antenna 40a is arranged at a portion adjacent to the protection hood 17 close to the rear end portion of the insertion section 9 (portion of head section 21c on front side apart from base section 21a), and the second antenna 40b is arranged at a portion near the battery 36 (position far from rear end portion of insertion section 9). Namely, these antennas 40a and 40b are arranged at both ends of the base section 21a in such a manner that the antennas 40a and 40b do not overlap a position in the base section 21a held by an operator (gripping section 18) if at all possible. Further, in the above-described substrate unit 30, a cable connecting the antennas to a wireless module substrate is arranged to form a substantially straight line with respect to the module substrate since the directivities of the antennas are affected depending on a direction along which the cable is arranged.

Since the first antenna 40a is arranged at the upper end portion and the second antenna 40b is arranged at the lower end portion of the base section 21a to be apart from each other, the wireless communication antennas can be separated from a body (hand) of an operator that blocks the electromagnetic waves as much as possible. Furthermore, it is possible to reduce a possibility that the electromagnetic waves from the wireless communication antennas are blocked by the portion in the endoscope operation section 8 where the metal components including the electric circuit are intensively provided to affect the communication. Therefore, this configuration can lead to the improvement of wireless communication performance in the endoscopic system 1 as compared with a conventional wireless endoscope, thereby effecting a smooth endoscopic procedure.

Moreover, the first antenna 40a is arranged at a position where at least a part of the first antenna 40a gets closer to the proximal end portion of the insertion section 9 than the bending operation section 15 when the gripping section 18 is held in such a manner that the insertion section 9 becomes substantially parallel to a floor. As a result, the first antenna 40a can be separated from the portion, e.g., a hand of an operator for the endoscope 2 or the metal components constituting the endoscope operation section 8 that inhibit the electromagnetic waves, thereby improving the wireless communication performance.

Additionally, in this case, the wireless communication antennas (the first and second antennas 40a and 40b) often face the reception antenna 7 side of the processing device 3 (at least a part of the first antenna 40a is close to the protection hood 17 on the front side apart from the base section 21a, and at least a part of the second antenna 40b is close to the front side apart from the portion where the metal body is arranged). Since the first and second antennas 40a and 40b are provided on the front side apart from the gripping section 18, thereby assuredly avoiding coming under the influence when the operator grips the gripping section 18. Therefore, the wireless communication antennas further contribute to the improvement in the electromagnetic wave communication performance. That is, it is possible to avoid of deterioration in response due to disconnection of communication during use of the endoscope 2, a picture quality displayed in the display device 4 can be prevented from being lowered, and an endoscopic image can be stably displayed in the display device 4.

It is to be noted that the wireless communication antennas (the first and second antennas 40a and 40b) are basically configured to transmit a wireless signal to the reception antennas 7 of the processing device 3, but the processing device 3 may have a function of transmitting the wireless signal and may be configured to send back the wireless signal including information indicative of a state "the wireless signal is correctly transmitted" as a response to the wireless communication antennas of the endoscope 2 through the reception antennas. That is, it is preferable to enable transmission and reception of the wireless signal between the first and second antennas 40a and 40b of the endoscope 2 and the processing device 3.

If an electromagnetic wave state is poor and the wireless communication cannot be sufficiently achieved between the endoscope 2 and the processing device 3, for example, as shown in FIG. 20, the processing device 3 may be set to display a frame 56 having a warning color such as an orange color in an outer frame of an endoscopic image displayed in the display device 4. Then, a user can be assuredly informed of the poor electromagnetic wave state without interrupting the endoscopic image.

According to this embodiment, it is possible to provide the endoscope 2 that has excellent griping properties/operability improved for an operator, reduces an influence of a human body of an operator who grips the endoscope operation section 8 or the metal body 44 in the endoscope operation section 8 including the electric circuit on directivities of the antennas 40a and 40b in the wireless communication, and has improved wireless communication performance.

As shown in FIG. 3 and FIG. 21, a battery accommodation section 57 is formed in a portion of the cylindrical body 52 in the base section 21a adjacent to the substrate unit 30 in the endoscope operation section 8. As shown in FIG. 21, the battery 36, e.g., a lithium ion rechargeable battery serving as a drive power supply of the endoscope 2 is detachably accommodated in the battery accommodation section 57. It is to be noted that the battery 36 is formed into, e.g., a substantially rectangular parallelepiped shape as shown in FIG. 22A and FIG. 22B.

Power from the battery 36 is supplied to the substrate unit 30 in the base section 21a and the light source unit 45. As shown in FIG. 21, a power supply cable 62 is extended to electric contacts 61 disposed in the battery accommodation section 57 so that the connection with the substrate unit 30 or the light source unit 45 in the base section 21a can be achieved to supply the power.

The battery accommodation section 57 includes a battery box 58 forming a space in which the battery 36 is mounted and accommodated and a battery lid 60 that closes a later-described battery accommodation opening 59 of the battery box 58. The battery accommodation opening 59 is formed at the lowermost portion of the base section 21a which is an end of a portion in which the substrate unit 30 is accommodated in a direction parallel to the central axis Cb of the gripping section 18. The battery lid 60 is configured to be opened via a hinge 63 in a state in which an operator vertically grips the base section 21a of the endoscope and to be closed by using a buckle mechanism 64. Further, in the buckle mechanism 64, a buckle lever 65 that is manually engaged or released by an operator is provided to form a smooth surface shape with a surface of a peripheral portion of the base section 21a in an engaged state.

The battery lid 60 is engaged with the cylindrical body 52 via the buckle mechanism 64 when arranging the battery 36 in the battery accommodation section 57 through the battery accommodation opening 59 and the battery accommodation opening 59 is closed to operate the endoscope 2.

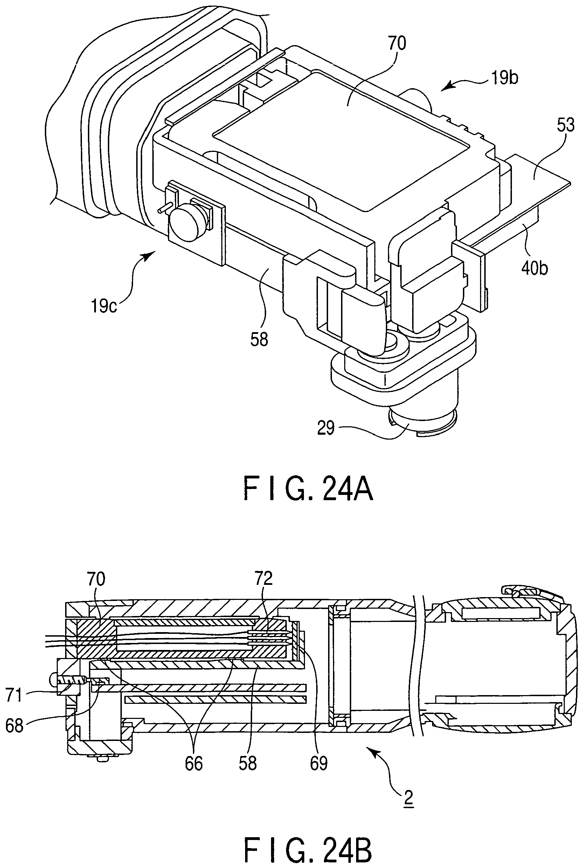

As shown in FIG. 21 and FIG. 24B, ribs 66 which accept corners of the battery 36 and regulate a position of the battery 36 in the battery box 58 are formed on the inner surface of the battery box 58. These ribs 66 support positions that are hardly affected by the expansion of the battery 36 so that the electric contacts 62 can be assuredly brought into contact with contacts 36a of the battery 36 even if the battery 36 is repeatedly used and the battery 36 thereby expands from a state indicated by a solid line to a state indicated by a broken line in FIG. 22B.

A claw 67 that can be engaged with the battery 36 having the substantially rectangular parallelepiped shape is formed near the battery accommodation opening 59. The claw 67 holds a housing of the battery 36 when the battery 36 is being accommodated in the battery accommodation section 57, and the engagement of the claw 67 with the battery 36 can be readily released by pushing down the claw 67 with a finger when removing the battery 36, thereby removing the battery 36 from the battery accommodation section 57.

Providing the claw 67 to the battery accommodation section 57 and further providing the battery lid 60 can prevent the battery 36 accommodated in the endoscope operation section 8 from coming off to stop supply of power when the battery lid 60 is accidentally opened during use of the endoscope. That is, possible interruption of a current in the substrate unit 30 at the moment of opening the battery lid 60 can be avoided.

Further, as described above, although the battery 36 expands to the state indicated by the broken line in FIG. 22B when it is used, since a degree of expansion is not clear, it is difficult to appropriately determine a dimension of the claw 67 provided to the battery accommodation section 57 so that it can catch the battery 36 without problem. Therefore, as shown in FIG. 21, the claw 67 is arranged at a position where it is caught by a side 36b having the least influence of the expansion of the battery 36 (see FIG. 22B) in the battery accommodation section 57.

Here, reference number 67a denotes a position at which the claw is provided in conventional examples. When the claw is provided at the position denoted by reference number 67a, the battery 36 may not be appropriately locked at the time of the expansion of the battery 36, or the battery 36 may be caught by the craw and thereby hardly removed from the battery box, but forming the claw at the position denoted by reference number 67 enables more assuredly locking the battery 36, and the difficulty in removing the battery 36 from the battery box can be eliminated even if the battery 36 expands.

Moreover, as shown in FIG. 21, a detection switch 68 which detects opening/closing of the battery lid 60 is formed near the battery accommodation opening 59 of the battery accommodation section 57. The detection switch 68 is pushed by a protrusion 60a on the battery lid 60 during an operation of the endoscope 2, and the detection switch 68 is set to display in the display device 4 a warning message indicating that the battery lid 60 is opened (not appropriately closed), stop display of an endoscopic image, or immediately execute shutdown processing for an operation of the endoscope 2 based on a regular procedure by using the electronic circuit provided in the substrate unit 30 when the battery lid 60 is opened and the pushing by the protrusion 60a is released. That is, in a state in which the detection switch 68 is not pushed by the protrusion 60a on the battery lid 60, a shutdown state is maintained, and the electric system of the endoscope 2 is not activated even if the third operation switch (power supply switch) 19c is operated. It is to be noted that, even if the battery lid 60 is opened, since the claw 67 prevents the battery 36 from immediately coming off the battery accommodation section 57, the battery 36 can be usually taken out after shutting down based on the regular procedure.

As shown in FIG. 17A, the bending operation lever 23, the bending operation section main body 50, and the griping section 18 are arranged from above in the direction vertical to the floor in the mentioned order, the first wireless antenna 40a is arranged above the gripping section 18, and the second wireless antenna 40b and the battery 36 that is the heaviest component in the endoscope operation section 8 are arranged below the gripping section 18, respectively. That is, when the endoscope 2 is held in such a manner that the insertion section 9 becomes substantially parallel to the floor, the battery 36 which is the heaviest component in the endoscope operation section 8 is arranged below the griping section 18. Therefore, the endoscope having the high stability and the low gravity center can be provided without lowering the wireless communication performance when an operator grips the gripping section 18 of the endoscope 2.

Additionally, since the endoscope 2 according to this embodiment is an endoscope adopting the wireless communication system without an external cable, the stability can be easily maintained even if the endoscope 2 is set itself up or set upright through a non-illustrated stand.

As shown in FIG. 23, various setting signals from an external write device (computer) 171 can be written or settings can be changed with respect to the circuit in the substrate unit 30 of the endoscope 2. Although the setting signals may be wirelessly written into the circuits in the substrate unit 30, it is preferable to provide a setting signal writing contact to the endoscope 2 and write the setting signals through a cable in order to further suppress increase in volume, complication of circuitry, and rise of price caused by additionally attaching to the substrate unit 30 a circuit which transmits/receives a wireless signal to/from the write device 171.

As shown in FIG. 21 and FIG. 24B, a signal writing pad 69 is formed in the battery accommodation section 57 in the endoscope 2 having water-tightness assured therein. The setting signal must be written in a state in which power is being supplied to the circuits in the substrate unit 30 like the use of the endoscope 2, and an instrument which supplies power from the power supply to the circuits in the substrate unit 30 and supplies the setting signal from the write device 171 is required.

For the above-described reason, a setting signal write instrument 70 formed to be accommodated in the battery accommodation section 57 is adopted as the instrument as shown in FIG. 23 and FIG. 24A. Contacts 72 (see FIG. 25A) that achieve electrical conduction with the signal writing pad 69 of the endoscope operation section 8 shown in FIG. 21 are provided to the setting signal write instrument 70, and contacts 73, as shown in FIG. 25A, through which a current from the power supply is supplied to the circuits in the substrate unit 30 are provided at substantially the same positions as the contacts 36a of the battery 36 attached to the battery box 58 of the endoscope 2 (see FIG. 25A and FIG. 25B).

As depicted in FIG. 23, the setting signal write instrument 70 is inserted into and used in the battery box 58 with the battery lid 60 being opened. Therefore, a protrusion 71 (see FIG. 24A and FIG. 24B) which pushes the detection switch 68 is provided to the setting signal write instrument 70 to avoid a situation that the shutdown processing of the endoscope 2 is executed to stop supply of the power since the detection switch 68 is not pushed by the battery lid 60. When such a setting signal write instrument 70 is used, the instrument which writes various setting signals can be attached to the battery accommodation section 57 without providing an additional structure to the endoscope 2.

As shown in FIG. 26 and FIG. 27, the processing device 3 includes a housing 3a mainly formed of a resin and metal panels. Various kinds of switches, e.g., a power supply switch 153, a wireless channel changeover switch 151, a test pattern color bar switch 154, and an image inversion switch 155, a channel indicator 152, antenna connectors 156a, 156b, and 156c to which the reception antennas 7a are detachably connected are provided to the outer side of the processing device 3. Respective circuit substrates 157 including a wireless circuit, an image processing circuit, and others are provided in the processing device 3.

That is, the wireless circuit in the processing device 3 is associated with a diversity antenna, and using two antennas, i.e., a main antenna and a sub-antenna enables efficiently transmitting/receiving wireless signals from the endoscope 2.

One antenna connector is provided on a front portion (front panel 158 side) designated by reference number 156a and two antenna connectors are provided on a rear portion (rear panel side) denoted by reference numbers 156b and 156c, respectively. The respective antenna connectors 156a, 156b, and 156c are provided on the endmost sides of the housing 3a of the processing device 3 in the width direction, and an interval required for assuring the performance of the two antennas 7 is set to be substantially equal to a width of the housing 3a, thereby achieving both a reduction in size of the housing 3a and securement of the transmission/reception performance. Furthermore, as positions at which additional antenna connectors are provided besides the antenna connectors 156a, 156b, and 156c, side surfaces of the housing 3a can be considered.

It is to be noted that a non-illustrated elastic member (e.g., rubber having hardness of 40 degrees) such as rubber or resin is provided to each of the antenna connectors 156a, 156b, and 156c to prevent each reception antenna from coming off even if vibration occurs in the processing device 3.

In this embodiment, for example, an antenna having a rod-like shape denoted by reference number 7a or 7b, a circular polarized antenna designated by reference number 160 can be attached to/detached from the antenna connector denoted by reference number 156a or 156b. The circular polarized antenna 160 includes a plug 160a connected to the antenna connector 156a or 156b and a cable 160b and can be installed at a portion apart from the processing device 3. Further, a rod-like hinge antenna designated by reference number 7a can be attached to the antenna connector denoted by reference number 156c. An angle of the hinge antenna 7a can be freely adjusted between a state in which the processing device 3 is installed on its side and long in the horizontal direction and a substantially upright state.

It is to be noted that the respective antenna connectors may have the same shape so that the hinge antenna 7a, the rod-like antenna 7b, or the circular polarized antenna 160 can be freely removed and reattached to the front or back side of the housing 3a in accordance with an installation space or an electromagnetic wave situation of the processing device 3.

Further, besides the three antenna connectors 156a, 156b, and 156c, an antenna substrate 161 may be included in the front panel 158 as shown in FIG. 27 so that members other than metals can be arranged around the antenna substrate 162 without a reduction in wireless performance, thereby preventing exterior parts of the antennas from protruding from the housing 3a of the processing device 3 as much as possible.

As a result, the built-in antenna 162, the rod-like antenna 7a, the hinge antenna 7b, and the circular polarized antenna 160 can be freely selected in accordance with an electromagnetic wave transmission/reception state, a use environment, installing positions of a cart, an examination desk, a rack, and others, and optimum arrangement and types of the reception antennas that do not obstruct various kinds of wiring lines in an examination room or system operations can be determined.

It is to be noted that the housing 3a of the processing device 3 is formed into a box shape by, e.g., combining metal panels for six surfaces. For example, a resin front panel 158 is provided as a front surface of the housing 3a. The front panel 158 is formed of a smooth curved surface 158a (e.g., a surface having approximately R900) to improve operability. Since using the curved surface 158a enables facilitating assembly of the housing 3a without loosing the operability, an operation plate 159 on which the respective indicators and the various operation switches 151, 153, 154, and 155 are integrally arranged is attached to the front panel 158.

Meanwhile, a configuration that the housing 3a of the processing device 3 can be easily opened is not allowed for the wireless device (the wireless device is not handled when opened). Therefore, to avoid disassembly of the housing 3a of the processing device 3, special screws 3b having such as a shape as depicted in FIG. 27 are utilized to dispose the respective panels so that these panels cannot be easily opened.

As shown in FIG. 28, when a screw 3c that fastens an earth cable disposed to, e.g., a terminal to obtain a main power supply from the outside is arranged coaxially with one of screws fastening (positioning) the housing panels of the processing device 3, an earth cable attachment space can be reduced, and the housing 3a can be minimized.

Moreover, each radiator hole 3d has a circular perforation shape having a shield effect against inhibiting electromagnetic waves from the outside and noise that leaks from the housing 3a to the outside. This shape can avoid a situation that one side has an antenna effect and electromagnetic wave inhibition occurs in the antenna substrate like an example where each radiator hole provided in the metal panel has a long hole shape.

Although one embodiment has been specifically described above with reference to the drawings, the present invention is not restricted to the foregoing embodiment, and it includes all embodiments which can be carried out.

### Reference Signs List

2···endoscope, 8···endoscope operation section (endoscope main body), 9···insertion section, 15···bending operation section, 17···protection hood, 18···gripping section, 19a···first operation switch, 19b···second operation switch, 19c---third operation switch, 21a···base section, 21b···curved section, 21c···head section, 23···bending operation lever, 29···vent mouth ring, 30···substrate unit, 40a···first antenna, 40b···second antenna, 41···indication lamp, 45···light source unit, 45a···illumination light source, 48···imaging cable, 52···cylindrical body, 52a···antenna accommodation section, 82···housing.

## Claims

1. An endoscope comprising:
an insertion section (9) which has a distal end portion, a rear end portion, and extended front and back directions defined by the distal end portion and the rear end portion, the distal end portion is adapted to be inserted into a body cavity;
an observation optical system which is provided in the insertion section (9), which acquires a picture of a subject;
a base section (21a) which has an upper end portion and a lower end portion;
a gripping section (18) which is provided between the upper end portion and the lower end portion of the base section (21a), which has a front side part facing to the front direction of the insertion section, and which is adapted to be gripped by an operator;
a curved section (21b) which is provided at the upper end portion of the base section (21a);
a head section (21c) which is coupled to the rear end portion of the insertion section (9) to protrude toward the base section (21a) at a predetermined angle, which is arranged between the curved section (21b) and the rear end portion of the insertion section;
a first antenna (40a) which is arranged between the rear end portion of the insertion section (9) and the front side part of the gripping section (18) and which is configured to be apart from a hand of the operator; and
a second antenna (40b) which is arranged at a position closer to the lower end portion of the base section (21a) and which is in front of the front side part of the gripping section (18) and which is configured to be apart from the hand of the operator, wherein
an image obtained by imaging the inside of the body cavity by the observation optical system is converted into a wireless signal, wherein the system is configured to enable transmission/reception of the wireless signal to/from the outside through the first (40a) and/or second antenna (40b).

2. The endoscope according to claim 1, wherein the predetermined angle is approximately 90 degrees to approximately 105 degrees.

3. The endoscope according to claim 1, wherein at least a part of each of the first (40a) and second antennas (40b) is arranged between the rear end portion side of the insertion section (9) and the gripping section (18) in the front and back directions.

4. The endoscope according to claim 1, wherein
the base portion includes electronic components which is configured to be electronically activated into the gripping portion, which are away from the first (40a) and second antennas (40b), and which is configured to be long in the vertical direction defined by the upper end portion and the lower end portion of the base section (21a) into the gripping portion,
the first antenna (40a) is arranged at a position closer to the rear end portion of the insertion section (9) than the electronic components in the vertical direction, and
the second antenna (40b) is arranged at a position farther from the rear end portion of the insertion section (9) than the electronic components in the vertical direction.

5. The endoscope according to claim 1, wherein
the base portion includes an inner metal frame configured to be long in the vertical direction defined by the upper end portion and the lower end portion of the base section (21a) into the gripping portion,
the first antenna (40a) is arranged at a position closer to the rear end portion of the insertion section (9) than the inner metal frame in the vertical direction, and
the second antenna (40b) is arranged at a position farther from the rear end portion of the insertion section (9) than the metal frame in the vertical direction.

6. The endoscope according to claim 1, wherein the first (40a) and second (40b) antennas are diversity antennas having different directivities.

## Patentansprüche

1. Endoskop, umfassend:
einen Einführabschnitt (9), der einen distalen Endabschnitt, einen hinteren Endabschnitt und verlängerte vordere und hintere Richtungen, die durch den distalen Endabschnitt und den hinteren Endabschnitt definiert sind, aufweist, wobei der distale Endabschnitt dazu ausgebildet ist, in einen Körperhohlraum eingeführt zu werden;
ein optisches Beobachtungssystem, das in dem Einführabschnitt (9) bereitgestellt ist, das ein Bild eines Subjekts erfasst;
einen Basisabschnitt (21a), der einen oberen Endabschnitt und einen unteren Endabschnitt aufweist;
einen Greifabschnitt (18), der zwischen dem oberen Endabschnitt und dem unteren Endabschnitt des Basisabschnitts (21a) bereitgestellt ist, der ein vorderes Seitenteil aufweist, das der vorderen Richtung des Einführabschnitts zugewandt ist und das ausgebildet ist, um von einem Bediener ergriffen zu werden;
einen gekrümmten Abschnitt (21b), der am oberen Endabschnitt des Basisabschnitts (21a) bereitgestellt ist;
einen Kopfabschnitt (21c), der mit dem hinteren Endabschnitt des Einführabschnitts (9) gekoppelt ist, um in einem vorgegebenen Winkel, der zwischen dem gekrümmten Abschnitt (21b) und dem hinteren Endabschnitt des Einführabschnitts angeordnet ist, in Richtung des Basisabschnitts (21a) hervorzustehen;
eine erste Antenne (40a), die zwischen dem hinteren Endabschnitt des Einführabschnitts (9) und dem vorderen Seitenteil des Greifabschnitts (18) angeordnet ist und die so ausgebildet ist, dass sie von einer Hand des Bedieners entfernt ist; und
eine zweite Antenne (40b), die an einer Position näher an dem unteren Endabschnitt des Basisabschnitts (21a) angeordnet ist und die sich vor dem vorderen Seitenteil des Greifabschnitts (18) befindet und die so ausgelegt ist, dass sie von der Hand des Bedieners entfernt ist, wobei
ein Bild, das durch Abbildung des Inneren des Körperhohlraums durch das optische Beobachtungssystem erhalten wird, in ein drahtloses Signal konvertiert wird, wobei das System ausgebildet ist, um die Sendung/den Empfang des drahtlosen Signals nach/von außen durch die erste (40a) und/oder zweite Antenne (40b) zu ermöglichen.

2. Endoskop nach Anspruch 1, wobei der vorgegebene Winkel etwa 90 Grad bis etwa 105 Grad beträgt.

3. Endoskop nach Anspruch 1, wobei mindestens ein Teil jeder der ersten (40a) und zweiten Antenne (40b) zwischen der hinteren Endabschnittsseite des Einführabschnitts (9) und dem Greifabschnitt (18) in die vordere und hintere Richtung angeordnet ist.

4. Endoskop nach Anspruch 1, wobei
der Basisabschnitt elektronische Elemente umfasst, der ausgebildet ist, um elektronisch in den Greifabschnitt aktiviert zu werden, die sich von der ersten (40a) und der zweiten Antenne (40b) entfernt befinden und der ausgebildet ist, um in vertikaler Richtung, die durch den oberen Endabschnitt und den unteren Endabschnitt des Basisabschnitts (21a) in den Greifabschnitt definiert wird, lang zu sein,
die erste Antenne (40a) an einer Position näher an dem hinteren Endabschnitt des Einführabschnitts (9) als die elektronischen Elemente in der vertikalen Richtung angeordnet ist und
die zweite Antenne (40b) an einer Position weiter von dem hinteren Endabschnitt des Einführabschnitts (9) als die elektronischen Elemente in der vertikalen Richtung entfernt angeordnet ist.

5. Endoskop nach Anspruch 1, wobei
der Basisabschnitt einen inneren Metallrahmen umfasst, der ausgebildet ist, um in der vertikalen Richtung, die durch den oberen Endabschnitt und den unteren Endabschnitt des Basisabschnitts (21a) in dem Greifabschnitt definiert ist, lang zu sein,
die erste Antenne (40a) in einer Position näher an dem hinteren Endabschnitt des Einführabschnitts (9) als der innere Metallrahmen in der vertikalen Richtung angeordnet ist und
die zweite Antenne (40a) in einer Position weiter von dem hinteren Endabschnitt des Einführabschnitts (9) als der Metallrahmen in der vertikalen Richtung entfernt angeordnet ist.

6. Endoskop nach Anspruch 1, wobei die erste (40a) und zweite (40b) Antenne Diversity-Antennen sind, die unterschiedliche Richtcharakteristiken aufweisen.

## Revendications

1. Endoscope comprenant :
une section d'insertion (9) qui a une partie d'extrémité distale, une partie d'extrémité arrière et des directions avant et arrière étendues définies par la partie d'extrémité distale et la partie d'extrémité arrière, la partie d'extrémité distale étant apte à être insérée dans une cavité corporelle ;
un système optique d'observation qui est disposé dans la section d'insertion (9), qui acquiert une image d'un sujet ;
une section de base (21a) qui a une partie d'extrémité supérieure et une partie d'extrémité inférieure ;
une section de saisie (18) qui est disposée entre la partie d'extrémité supérieure et la partie d'extrémité inférieure de la section de base (21a), qui a une partie côté avant tournée dans la direction avant de la section d'insertion, et qui est apte à être saisie par un opérateur ;
une section courbe (21b) qui est disposée au niveau de la partie d'extrémité supérieure de la section de base (21a) ;
une section de tête (21c) qui est couplée à la partie d'extrémité arrière de la section d'insertion (9) pour faire saillie vers la section de base (21a) à un angle prédéterminé, qui est agencée entre la section courbe (21b) et la partie d'extrémité arrière de la section d'insertion ;
une première antenne (40a) qui est agencée entre la partie d'extrémité arrière de la section d'insertion (9) et la partie côté avant de la section de saisie (18) et qui est configurée pour être espacée d'une main de l'opérateur ; et
une seconde antenne (40b) qui est agencée à une position plus proche de la partie d'extrémité inférieure de la section de base (21a) et qui est devant la partie côté avant de la section de saisie (18) et qui est configurée pour être espacée de la main de l'opérateur,
une image obtenue par imagerie de l'intérieur de la cavité corporelle par le système optique d'observation étant convertie en un signal sans fil, le système étant configuré pour permettre l'émission/réception du signal sans fil vers/depuis l'extérieur par l'intermédiaire de la première (40a) et/ou seconde antenne (40b).

2. Endoscope selon la revendication 1, dans lequel l'angle prédéterminé est d'approximativement 90 degrés à approximativement 105 degrés.

3. Endoscope selon la revendication 1, dans lequel au moins une partie de chacune des première (40a) et seconde antennes (40b) est agencée entre le côté partie d'extrémité arrière de la section d'insertion (9) et la section de saisie (18) dans les directions avant et arrière.

4. Endoscope selon la revendication 1, dans lequel :
la partie de base comprend des composants électroniques qui sont configurés pour être activés électroniquement dans la partie de saisie, qui sont à distance des première (40a) et seconde antennes (40b), et qui est configurée pour être longue dans la direction verticale définie par la partie d'extrémité supérieure et la partie d'extrémité inférieure de la section de base (21a) jusque dans la partie de saisie,
la première antenne (40a) est agencée à une position plus proche de la partie d'extrémité arrière de la section d'insertion (9) que les composants électroniques dans la direction verticale, et
la seconde antenne (40b) est agencée à une position plus éloignée de la partie d'extrémité arrière de la section d'insertion (9) que les composants électroniques dans la direction verticale.

5. Endoscope selon la revendication 1, dans lequel :
la partie de base comprend un cadre métallique interne configuré pour être long dans la direction verticale définie par la partie d'extrémité supérieure et la partie d'extrémité inférieure de la section de base (21a) jusque dans la partie de saisie,
la première antenne (40a) est agencée à une position plus proche de la partie d'extrémité arrière de la section d'insertion (9) que le cadre métallique interne dans la direction verticale, et
la seconde antenne (40b) est agencée à une position plus éloignée de la partie d'extrémité arrière de la section d'insertion (9) que le cadre métallique dans la direction verticale.

6. Endoscope selon la revendication 1, dans lequel les première (40a) et seconde (40b) antennes sont des antennes à réception en diversité ayant des directivités différentes.
